# EUROPEAN PATENT SPECIFICATION

(11) **EP 1 392 779 B1**
(45) Date of publication and mention of the grant of the patent: **22.04.2009**
(21) Application number: 01996581.3
(22) Date of filing: 13.11.2001
(51) Int. Cl.: C09D 4/00, C07C 49/80

(54) **FLUORINATED-PHOTOINITIATORS IN DUAL CURE RESINS**
FLUORIERTE PHOTOINITIATOREN IN DUAL-CURE-HARZEN
PHOTOAMORCEURS FLUORES UTILISES DANS LES RESINES A DOUBLE POLYMERISATION

(30) Priority: 20.11.2000 EP 00811098
(43) Date of publication of application: 03.03.2004
(73) Proprietor: Ciba Holding Inc., 4057 Basel (CH)
(72) Inventor: BAUDIN, Gisèle, CH-4123 Allschwil (CH); JUNG, Tunja, 79618 Rheinfelden-Herten (DE); HÜSLER, Rinaldo, CH-4055 Basel (CH)
(86) International application number: PCT/EP2001/013130
(87) International publication number: WO 2002/040602

(56) References cited:
- WO-A-93/12150
- SCHULTZ, J. W.; CHARTOFF, R. P.: "Photopolymerization of Nematic Liquid Crystal monomers for structural applications: Linear Viscoelastic Behavior and cure effects." J. POLYM. SCI.: PART B, POLYM. PHYS, vol. 36, 1998, pages 1081-1089, XP002191239

## Description

The present invention relates to a process for the preparation of scratch-resistant coatings by dual cure of compositions wherein surface active fluorinated photoinitiators are present. The invention also relates to a process for concentrating a surface-active photoinitiator in the surface of coatings, to novel surface active fluorinated photoinitiators, compositions wherein the novel surface active fluorinated photoinitiators are present, and to a method for improving the flow of a curable composition on substrates.

The cure of polymer coatings by exposure to ultraviolet (UV) radiation offers certain advantages of speed, cost effectiveness and avoidance of the need of solvents. Photocurable coating compositions are usually comprised of three components: polymerizable monomers, a photoinitiator and optionally additives, such as pigments.

WO 93/12150 discloses surface active benzoyl compounds wherein at least one non-polar fluorinated alkyl group is present and their use as photoinitiators in UV-curable polymer compositions. An example of these surface active benzoyl compounds is represented by the formula 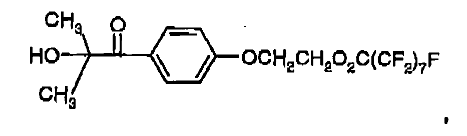 wherein the non-polar fluorinated alkyl group is located In 4-position of the phenyl ring. WO 93/12150 also discloses other photoinitiator molecules wherein the fluorinated alkyl group is present in different positions of the benzoyl moiety, e.g. in a position α- or β- to the carbonyl group in the aliphatic side chain. In the alternative, two non-polar fluorinated alkyl groups may also be present, e.g. In 4-position of the phenyl ring and in the aliphatic side chain. The photoinitiators of the present invention have the perfluorinated side chains connected to the photoinitiating moiety via a linking unit bearing S-, Si-, or N-atoms.

US 5922473 describes dual cure system comprising a thermal initiator, such as a peroxide, along with a UV initiator. The claimed photoinitiators are not disclosed.

WO98/00456 describes a curing process combining thermal and UV curing. By means of this curing process it is possible, using compositions comprising (A) a coating system based on a polyacrylate polyol or polyester polyol with melamine, or a polyacrylate polyol and/or polyester polyol with a blocked or unblocked polyisocyanate, or a carboxyl-, anhydride- or amino-functional polyester and/or polyacrylate with an epoxy-funotional polyester or polyacrylate, (B) an OH-, NH2-, COOH-, epoxy- or NCO-functional resin containing, in addition, at least one ethylenically unsaturated double bond, which is separated from the functional group by a spacer group, and (C) at least one photoinitiator, to obtain coatings having good surface properties. The claimed photoinitiators are not disclosed.

Thermal postcuring has also been described by J.W. Schulz and R.P. Chartoff in J. Polymer Science, Part B, Polymer Phys., Vol. 36, 1998, pages 1081-1089 In this reference postcuring is applied in a composition comprising a commercial ketone photoinitiator,

Despite its wide use, certain disadvantages of UV-curing technology are known. According to Concise Encyclopedia of Polymer Science and Engineering (J. I. Kroschwitz Editor), John Wiley & Sons 1990, ISBN 0-471-51253-2, cf. the entry "photopolymerization" on page 727: "Limitations of UV-curing technology include the utilization of oddly shaped substrates and the curing of opaque coatings. Weatherability or durability requires special consideration, since conventional light stabilizers tend to Interfere with the UV-curing process. Latent stabilizers that are activated by light absorption may alleviate this problem".

In order to overcome these problems and to lower emissions, save energy and to obtain etch and mar resistant clearcoats, the coating industry is exploring new curing and/or application technologies. A proposed route as disclosed in the U.S. Patent Specification 5,922,479 consists of a combination of thermosetting coating with UV-cure. The UV-cure may take place before, during or after thermal curing ("dual cure"). In order to improve the mechanical properties of the coatings against mechanical impacts, such as scratching, dual cure technology is now a preferred method in coating technology. There is an increasing need for suitable photoinitiators useful for the dual cure of polymer compositions.

Surprisingly it has been found that surface active benzoyl compounds wherein at least one non-polar fluorinated alkyl group is present, are useful in the dual cure of polymer compositions. The fluorinated alkyl group can be present at different positions in the benzoylmolecule, thus distinguishing between embodiment 1, embodiment 2 and embodiment 3. The concentration of these photoinitiators is increased at the surface in top layers of coatings which improves their mechanical properties.

The present invention relates to a process for the preparation of coatings which is characterized in that a composition comprising
a) at least one polymerization initiator of the formula 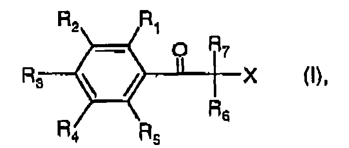 wherein in embodiment (1)
   - X: represents the terminal groups RₐO- or RₐR_{b}N-; and one or two of
   - R₁ - R₅: represent substituents selected from the group consisting of -R_{c}-Y-, R_{c}-Y-(CH₂)ₓ-, R_{c}-(CH₂)ₓ-Y-, R_{c}-Y-(CH₂)_{y}-O-, Rₒ-Y-(CH₂)_{y}-S- and
   R_{c}-Y-(CH₂)_{y}-NRₐ-; and the other R₁ - R₅ represent hydrogen or substituents selected from the group consisting of C₁-C₄alkyl, hydroxy, hydroxy-C₂-C₄alkyl, C₁-C₄alkoxy, hydroxy-C₂-C₄alkoxy, halogen, amino and di-C₁-C₄alkylamino; or
   wherein in embodiment (2)
   - X: represents a terminal group selected from the group consisting of R_{c}-Y₁-, R_{c}-CH₂)ₓ-Y₁-, R_{c}-Y-(CH₂)_{y}-O- and R_{c}-Y-(CH₂)_{y}-NR₈-; and
   - R₁ - R₅: represent hydrogen or substituents selected from the group consisting of C₁-C₄alkyl, hydroxy, hydroxy-C₂-C₄alkyl, C₁-C₄alkoxy, hydroxy-C₂-C₄alkoxy, halogen, amino and di-C₁-C₄alkylamino; or
   wherein in embodiment (3)
   - X: represents a terminal group selected from the group consisting of R_{c}-Y₁-, R_{c}-(CH₂)ₓ-Y₁-, R_{c}-Y-(CH₂)_{y}-O- and R_{c}-Y-(CH₂)_{y}-NR₈-; and one or two of
   - R₁ - R₅: represent substituents selected from the group consisting of R_{c}-Y-, Rₒ-Y-(CH₂)ₓ-, R_{c}-(CH₂)ₓ-Y-, R_{c}-Y-(CH₂)_{y}-O-, R_{c}-Y-(CH₂)_{y}-S- and R_{c}-Y-(CH₂)_{y}NR₈-;and the other R₁ - R₅ represent hydrogen or substituents selected from the group consisting of C₁-C₄alkyl, hydroxy, hydroxy-C₂-C₄alkyl, C₁-C₄alkoxy, hydroxy-C₂C₄alkoxy, halogen, amino and di-C₁-C₄alkylamino; and
   wherein in embodiment (1), (2) and (3)
   - R₆ and R₇: independently of one another represent C₁-C₁₂alkyl, C₂-C₈alkenyl, C₅-C₈cycloalkyl or phenyl-C₁-C₃alkyl; or R₆ and R₇ together represent C₂-C₈alkylene, C₃-C₉oxaalkylene or C₃-C₉-azaalkylene;
   - Rₐ and R_{b}: independently of one another represent hydrogen, C₁-C₁₂alkyl or C₂-C₆alkenyl; or Rₐ and R_{b} together represent C₄-C₅alkylene and together with the nitrogen atom to which they are bonded form a 5- or 6-membered ring, which may be interrupted by -O- or by -N-R₈-;
   - R_{c}: represents a linear or branched terminal chain ZCF₂(-O-C₂F₄)ₚ-(CF₂)_{q}-, wherein Z represents -H or -F; one of p and q represents a numeral from zero to twenty and the other one a numeral from one to twenty;
   - x: represents a numeral from one to ten;
   - y: represents a numeral from two to ten;
   - Y: represents a bivalent substituent selected from the group consisting of -S-, -NR₈-, -Si(R₉)₂-, -Si(R₈)₂-O-, -O-Si(R₈)₂-O- and -O-Si(R₈)₂-(CH₂)ₓ-;
   - Y₁: represents a bivalent substituent selected from the group consisting of -NR₈-, -O-Si(R₉)₂, -O-Si(R₉)₂-O- and -O-Si(R₉)₂-(CH₂)ₓ-;
   - R₈: represents -H or C₁-C₁₂alkyl; and
   - R₉: represents C₁-C₁₂alkyl or phenyl;
   and
b) polymerizable, ethylenically unsaturated compounds; and, optionally, as further components; a thermally crosslinkable compound; and/or further additives; and/or additional photoinitiators;
   is applied to a support and cured by irradiation with electromagnetic radiation of a wavelength from 200 nm up to the IR domain combined with prior, simultaneous or subsequent thermal curing.

Within the context of the description of the present invention, the general expressions and terms recited herein-above and below preferably are defined as follows:
The term coating defines layers of polymerisates applied to suitable substrates, such as metals, glass, ceramics, wood, paper, plastics, textiles or others.

The term composition defines any homogeneous or heterogeneous mixture which is applicable to a suitable support or substrate. The composition comprises as components
a) at least one polymerization initiator of the formula (I); and
b) polymerizable, ethylenically unsaturated compounds; and, optionally, as further components;
c) thermally crosslinkable compound; and/or
d) further additives; and/or
e) additional photoinititors.

### Component a) Polymerization initiators

The formula I comprises within its scope of the definitions of R₁ - R₅ and the terminal group -X three distinct structural embodiments of polymerization initiators:
According to the **embodiment (1),** the terminal group X is short chained. At least one non-polar group is a substituent of the phenyl ring of the polymerization initiator (I), preferably in 4-position (para).
According to the **embodiment (2),** the terminal group X is non-polar. R₁ - R₅ in the polymerization initiator (I) represent hydrogen or short chained substituents.
According to the **embodiment (3),** the terminal group X is non-polar. At least one additional non-polar group is a substituent of the phenyl ring of the polymerization initiator (I), preferably in 4-position (para).

**According to the embodiment (1),** X represents the terminal groups RₐO- or RₐR_{b}N-.
In these terminal groups Rₐ and R_{b} independently of one another represent hydrogen, C₁-C₁₂alkyl or C₂-C₆alkenyl;or
Rₐ and R_{b} together also represent C₄-C₅alkylene and together with the nitrogen atom to which they are bonded form a 5- or 6-membered heterocycle which may be interrupted by the additional heteroatom -O- or the group -N-R₈-, e.g. Rₐ and R_{b} together form a morpholinyl, piperidinyl or piperazinyl ring.
Preferred terminal groups X are, for example, hydroxy, di-C₁-C₄alkylamino, e.g. dimethylamino or diethylamino, or morpholinyl.
According to the embodiment (1), one or two of R₁ - R₅ represent substituents selected from the group consisting of -R_{c}-Y-, R_{c}-Y-(CH₂)ₓ-, F_{c}-(CH₂)ₓ-Y-, R_{c}-Y-(CH₂)_{y}-O-, Rₒ-Y-(CH₂)_{y}-Sand R_{c}-Y-(CH₂)_{y}NR₈-.
R_{c} represents a linear or branched terminal chain ZCF₂(-O-C₂F₄)ₚ-(CF₂)_{q}-,
wherein
Z represents -H or -F;
one of p and q represents a numeral from zero to twenty and the other one a numeral from one to twenty;

Preferred groups R_{c} contain 3-18 carbon atoms and the maximum number or fluorine substituents (perfluoroalkyl) and are linear (attachment in 1-position), e.g. heptafluoropropyl, nonafluorobutyl, undecafluoropentyl, tridecafluorohexyl, pentadecafluoroheptyl, heptadecafluorooctyl or nonadecafluorononyl.

Y in the combined groups R_{c}-Y-, R_{c}-Y-(CH₂)ₓ-, R_{c}-(CH₂)ₓ-Y-, R_{c}-Y-(CH₂)_{y}-O-, R_{c}-Y-(CH₂)_{y}-Sand R_{c}-Y-(CH₂)_{y}-NR₈- preferably represents a bivalent substituent selected from the group consisting of -S-, -NR₆-, -Si(R₉)₂-, -Si(R₉)₂-O-, -O-Si(R₉)₂-O- and -O-Si(R₉)₂-(CH₂)ₓ-.
Y₁ in the combined groups present in the terminal groups R_{c}-Y₁- and R_{c}-(CH₂)ₓ-Y₁- preferably represents a bivalent substituent selected from the group consisting of -NR₈-, -O-Si(R₉)₂-, -O-Si(R₉)₂-O- and -O-Si(R₉)₂-(CH₂)ₓ-.
In these combined groups Rₐ represents -H or C₁-C₁₂alkyl, preferably C₁-C₄-alkyl, e.g. methyl or ethyl.
R₉ substituting the silicon atom represents C₁-C₈alkyl or phenyl, e.g. methyl, ethyl or phenyl.
The bivalent substituent Y and Y₁ in the combined group R_{c}-Y- is preferably -S- and -O-Si(R₉)₂-(CH₂)ₓ-;
The combined group R_{c}-Y- preferably 1-heptafluoropropylthio, 1-nonafluorobutylthio, 1-undecafluoropentylthio, 1-tridecafluorohexylthio, 1-pentadecafluoroheptylthio or 1-nonadecafluorononylthio.

In the combined group R_{c}-Y-(CH₂)ₓ- the index x preferably represents a numeral from one to four and R_{c} preferably contains 3-12 carbon atoms and the maximum number of fluorine atoms. Such combined groups are, for example, 1-heptafluoropropylthiomethyl, 2-(1-heptafluoropropylthio)-ethyl, 1-nonafluorobutylthiomethyl, 2-(1-nonafluorobutylthio)-ethyl, 1-undecafluoropentylthiomethyl or 2-(1-undecafluoropentylthio)-ethyl.

In the combined group R_{c}-(CH₂)ₓ-Y- the index x preferably represents a numeral from one to four and R_{c} preferably contains 3-12 carbon atoms and the maximum number of fluorine atoms. Such groups are, for example, 3,3,4,4,5,5,6,6,7,7,8,8,8-tridecafluorooctylthio, 3,3,4,4,5,5,6,6,7,7,8,8,9,9,9-pentadecafluorononylthio, 3,3,4,4,5,5,6,6,7,7,8,8,9,9,10,10,10-heptadecafluorodecylthio, 3,3,4,4,5,5,6,6,7,7,8,8,9,9,10,10,11,11,11-nonadecafluoroundecyl-thio or 3,3,4,4,5,5,6,6,7,7,8,8,9,9,10,10,11,11,12,12,12-heneicosafluorododecylthio.

In the combined group R_{c}-Y-(CH₂)_{y}-O- the index y preferably represents a numeral from two to four, Y Is preferably -O- and R_{c} is preferably the acyl group of a perfluorinated linear C₃-C₁₆alkylcarbonic acid, e.g. hoptafluorobutanoyl, nonafluoropentanoyl, undecafluoro-hexanoyl, tridecafluoroheptanoyl, pentadecafluorooctanoyl or heptadecafluorononoyl. Such groups are, for example, 2-heptafluorobutanoyloxyethoxy, 2-nonafluoropentanoyloxyethoxy, 2-undecafluorohexanoyloxyethoxy, 2-tridecafluoroheptanoyloxyethoxy, 2-pentadeca-fluorooctanoyloxyethoxy or 2-heptadecafluorononoyloxyethoxy.
Another embodiment relates to the combined groups R_{c}-Y-, R_{c}-Y₁- and R_{c}-Y-(CH₂)_{y}-O-,
wherein the index y preferably represents a numeral from two to four and Y and Y₁ represent a bivalent silyl group. The bivalent silyl group in Y is selected from the group consisting of -Si(R₉)₂-, -Si(R₉)₂-O-, -O-Si(R₉)₂-O- and -O-Si(R₉)₂-(CH₂)ₓ-. The bivalent silyl group in Y₁ is selected from the group consisting of -O-Si(R₉)₂, -O-Si(R₉)₂-O- and -O-Si(R₉)₂-(CH₂)ₓ-, R₉ preferably represents methyl, ethyl or phenyl, R_{c} is,. for example, tridecafluorohexyl, pentadecafluoroheptyl, heptadecafluorooctyl, nonadecafluorononyl or heneicosafluorodecyl. Such groups are, for example 2-[(3,3,4,4,5,5,6,6,7,7,8,8,8-tridecafluorooctyl)-dimethylsilyl-oxy]-ethoxy, 2-[(3,3,4,4,5,5,6,6,7,7,8,8,9,9,9-pentadecafluorononyl)-dimethylsilyloxy]-ethoxy, 2-[(3,3,4,4,5,5,6,6,7,7,8,8,9,9,10,10,10-heptadecafluorodecyl)-dimethylsilyloxy]-ethoxy, 2-[(3,3,4,4,5,5,8,6,7,7,8,8,9,9,10,10,11,11,11-nonafluoroundecyl)-dimethylsilyoxy]-ethoxy, or 2-[(3,3,4,4,5,5,6,6,7,7,8,8,9,9,10,10,11,11,12,12,12-heneicosafluorododecyl)-dimethylsilyl-oxy]-ethoxy.
The structure of the combined groups R_{c}-Y-(CH₂)_{y}-S- and R_{c}-Y-(CH₂)_{y}-NR₈- is analogous to the combined group R_{c}-Y-(CH₂)_{y}-O-, wherein R_{c}, the index y and -Y- are as defined above. R₈ is preferably C₁-C₄-alkyl, e.g. methyl or ethyl.
In the polymerization initiator (I) the other R₁ - R₅ represent hydrogen or substituents selected from the group consisting of C₁-C₄alkyl, i.e. methyl, ethyl, n-propyl, isopropyl, n-butyl, isobutyl or tert.-butyl, hydroxy, hydroxy-C₂-C₄alkyl, e.g. 2-hydroxyethyl, C₁-C₄alkoxy, e.g. methoxy or ethoxy, hydroxy-C₂-C₄alkoxy, e.g. 2-hydroxyethoxy, halogen, e.g. fluoro, chloro or bromo, amino and di-C₁-C₄alkylamino, e.g. dimethylamino or diethylamino.

**According to the alternative embodiment (2),** the terminal group X in the polymerization initiator (I) is selected from the group consisting of R_{c}-Y₁-, R_{c}-(CH₂)ₓ-Y₁-, R_{c}-Y-(CH₂)_{y}-O- and R_{c}-Y-(CH₂)_{y}-NR₈-, particularly a terminal group selected from the group consisting of R_{c}-Y₁-, R_{c}-(CH₂)ₓ-Y₁- and R_{c}-Y-(CH₂)_{y}-O-. These combined groups are analogous to the corresponding groups defined above with regard to the embodiment (1). The definitions of R_{c}, Y₁, Y, x and y are also analogous.
According to the embodiment (2), R₁ - R₅ in the polymerization initiator (I) represent hydrogen or substituents selected from the group consisting of C₁-C₄alkyl, i.e. methyl, ethyl, n-propyl, isopropyl, n-butyl, isobutyl or tert.-butyl, hydroxy, hydroxy-C₂-C₄alkyl, e.g.2-hydroxyethyl, C₁-C₄alkoxy, e.g. methoxy or ethoxy, hydroxy-C₂-C₄alkoxy, e.g. 2-hydroxyethoxy, halogen, e.g. chloro or bromo, amino and di-C₁-C₄alkylamino, e.g. dimethylamino or diethylamino.

**According to the alternative embodiment (3),** the terminal group X in the polymerization initiator (I) is selected from the group consisting of R_{c}-Y₁-, R_{c}-(CH₂)ₓ-Y₁-, R_{c}-Y-(CH₂)_{y}-O- and R_{c}-Y-(CH₂)_{y}-NR₈-, particularly a terminal group selected from the group consisting of R_{c}-Y₁-, R_{c}-(CH₂)ₓ-Y₁- and R_{c}-Y-(CH₂)_{y}-O-. These combined groups are analogous to the corresponding groups defined above with regard to embodiment (1). The definitions of R_{c}, Y₁, Y, x and y are also analogous.
According to the embodiment (3), one or two of R₁ - R₆ represent substituents selected from the group consisting of R_{c}-Y-, R_{c}-Y-(OH₂)ₓ-, R_{c}-(CH₂)ₓ-Y-, R_{c}-Y-(CH₂)_{y}-O-, R_{c}-Y-(CH₂)_{y}-Sand R_{c}-Y-(CH₂)_{y}-NR₈-. These combined groups are analogous to the corresponding groups defined above with regard to embodiment (1). The definitions of R_{c}, Y, x and y are also analogous.
The other R₁ - R₅ represent hydrogen or substituents selected from the group consisting of C₁-C₄alkyl, i.e. methyl, ethyl, n-propyl, isopropyl, n-butyl, isobutyl or tert.-butyl, hydroxy, hydroxy-C₂-C₄alkyl, e.g. 2-hydroxyethyl, C₁-C₄alkoxy, e.g. methoxy or ethoxy, hydroxy-C₂-C₄alkoxy, e.g. 2-hydroxyethoxy, halogen, e.g. chloro or bromo, amino and di-C₁-C₄alkylamino, e.g. dimethylamino or diethylamino.
According to the embodiments (1), (2) and (3), R₆ and R₇ are, for example, C₁-C₄alkyl, especially methyl. Preferably R₆ and R₇ are methyl or together are C₃-C₈alkylene; in particular, together with the carbon atom to which they are bonded they form a cyclohexyl ring, or R₆ is C₁-C₄alkyl, especially ethyl, and R₇ is allyl or benzyl.
R₆ and R₇ are preferably identical, especially C₁-C₄alkyl, especially methyl or ethyl.
The compounds of formula I can be prepared in analogy to the process described in *WO 93*/*12150*.

A number of compounds of the formula I are novel and are also subject matter of the present invention.
The polymerization initiators (I), have the uniform concept in common that they are present as component a) in the composition defined above. They are used according to the process of the present invention in the curing of free-radically polymerizable systems by a combination of thermal and UV-crosslinking processes ("dual cure"). It is intended to concentrate the photoinitiator at the surface of the composition to achieve a good surface cure. This is provided with suitable photoinitiators of the formula I. In certain cases it may be advantageous to use mixtures of two or more of the photoinitiators of the formula I; for example it may be advantageous to use mixtures formed directly during their preparation. According to the process of the invention, improved surface properties are obtained by using the photoinitiators in compositions cured by a combination of thermosetting coating with UV-cure ("dual cure").

### Component b)

Polymerizable, ethylenically unsaturated compounds contain one or more olefinic double bonds. They may be of low molecular weight (monomeric) or higher molecular weight (oligomeric). Examples of monomers having one double bond are alkyl and hydroxyalkyl acrylates and methacrylates, such as methyl, ethyl, butyl, 2-ethylhexyl and 2-hydroxyethyl acrylate, isobornyl acrylate and methyl and ethyl methacrylate. Further examples thereof are acrylonitrile, acrylamide, methacrylamide, N-substituted (meth)acrylamides, vinyl esters, such as vinyl acetate, vinyl ethers, such as isobutyl vinyl ether, styrene, alkyl- and halo-styrenes, N-vinylpyrrolidone, vinyl chloride and vinylidene chloride.
Examples of monomers having more than one double bonds are ethylene glycol diacrylate, propylene glycol diacrylate, neopentyl glycol diacrylate, hexamethylene glycol diacrylate and bisphenol A diacrylate, 4,4'-bis(2-acryloyloxyethoxy)diphenylpropane, trimethylolpropane tri-acrylate, pentaerythritol triacrylate or tetraacrylate, vinyl acrylate, divinyl benzene, divinyl succinate, diallyl phthalate, triallyl phosphate, triallyl isocyanurate and tris(2-acryloylethyl) isocyanurate.
Examples of higher molecular weight (oligomeric) ethylenically unsaturated compounds are acrylated epoxy resins, acrylated or vinyl-ether- or epoxy-group-containing polyesters, polyurethanes and polyethers. Further examples of these unsaturated oligomers are unsaturated polyester resins, which are obtainable from maleic acid, phthalic acid and one or more diols. These oligomers have molecular weights of approximately from 500 to 3000. Vinyl ether monomers and oligomers, and maleate-terminated oligomers having polyester, polyurethane, polyether, polyvinyl ether and epoxy main chains can also be used. In particular, combinations of vinyl-ether-group-carrying oligomers and polymers, as described in WO 90/01512, are suitable. Also suitable are copolymers of monomers functionalized with vinyl ether and maleic acid. Such unsaturated oligomers can also be referred to as prepolymers.
Functionalized acrylates are also suitable. Examples of suitable monomers forming the backbone (the base polymer) of such functionalized acrylate and methacrylate polymers are, for example, acrylate, methacrylate, methyl methacrylate, ethyl acrylate, ethyl methacrylate, n-butyl acrylate, n-butyl methacrylate, isobutyl acrylate, isobutyl methacrylate, 2-ethylhexyl acrylate, 2-ethylhexyl methacrylate, etc.. In addition, suitable amounts of functional monomers are copolymerized during the polymerization so as to obtain the functional polymers. Acid-functionalized acrylate or methacrylate polymers are obtained using acid-functional monomers, such as acrylic acid and methacrylic acid. Hydroxy-functional acrylate or methacrylate polymers are produced from hydroxy-functional monomers, such as 2-hydroxyethyl methacrylate, 2-hydroxypropyl methacrylate and 3,4-dihydroxybutyl methacrylate. Epoxy-functionalized acrylate or methacrylate polymers are obtained using epoxy-functional monomers, such as glycidyl methacrylate, 2,3-epoxybutyl methacrylate, 3,4-epoxybutyl methacrylate, 2,3-epoxycyclohexyl methacrylate, 10,11-epoxyundecyl methacrylate, etc.. It is also possible to prepare isocyanate-functionalized polymers from isocyanate-functionalized monomers, such as meta-isopropenyl-α,α-dimethylbenzyl isocyanate.
Especially suitable are, for example, esters of ethylenically unsaturated mono- or poly-functional carboxylic acids and polyols or polyepoxides, and polymers having ethylenically unsaturated groups in the chain or in side groups, such as unsaturated polyesters, poly-amides and polyurethanes and copolymers thereof, alkyd resins, polybutadiene and butadiene co-polymers, polyisoprene and isoprene copolymers, polymers and copolymers having (meth)-acrylic groups in side chains, and mixtures of one or more such polymers.
Examples of suitable mono- or poly-functional unsaturated carboxylic acids are acrylic acid, methacrylic acid, crotonic acid, itaconic acid, cinnamic acid, maleic acid and fumaric acid and unsaturated fatty acids, such as linolenic acid or oleic acid. Preference is given to acrylic acid and methacrylic acid.
Saturated di- or poly-carboxylic acids in admixture with unsaturated carboxylic acids may, however, also be used. Examples of suitable saturated di- or poly-carboxylic acids include, for example, tetrachlorophthalic acid, tetrabromophthalic acid, phthalic acid anhydride, adipic acid, tetrahydrophthalic acid, isophthalic acid, terephthalic acid, trimellitic acid, heptanedi-carboxylic acid, sebacic acid, dodecanedicarboxylic acid, hexahydrophthalic acid, etc..
Suitable polyols are aromatic and especially aliphatic and cycloaliphatic polyols. Examples of aromatic polyols are hydroquinone, 4,4'-dihydroxydiphenyl, 2,2-di(4-hydroxyphenyl)-propane, novolaks and resoles. Examples of polyepoxides are those based on the polyols mentioned, especially aromatic polyols and epichlorohydrin. Also suitable as polyols are polymers and copolymers containing hydroxyl groups in the polymer chain or in side groups, such as polyvinyl alcohol and copolymers thereof or polymethacrylic acid hydroxyalkyl esters or copolymers thereof. Further suitable polyols are oligoesters having hydroxyl terminal groups.
Examples of aliphatic and cycloaliphatic polyols are alkylenediols having preferably from 2 to 12 carbon atoms, such as ethylene glycol, 1,2- or 1,3-propanediol, 1,2-, 1,3- or 1,4-butanediol, pentanediol, hexanediol, octanediol, dodecanediol, diethylene glycol, triethylene glycol, polyethylene glycols having molecular weights of preferably from 200 to 1500, 1,3-cyclopentanediol, 1,2-, 1,3- or 1,4-cyclohexanediol, 1,4-dihydroxymethylcyclohexane, glycerol, tris(β-hydroxy-ethyl)amine, trimethylolethane, trimethylolpropane, pentaerythritol, dipentaerythritol and sorbitol.
The polyols may be partially or fully esterified by one or by different unsaturated carboxylic acid(s), it is possible for the free hydroxyl groups in partial esters to have been modified, for example etherified, or esterified with other carboxylic acids.

Examples of these esters are: trimethylolpropane triacrylate, trimethylolethane triacrylate, trimethylolpropane trimethacrylate, trimethylolethane trimethacrylate, tetramethylene glycol dimethacrylate, tri-ethylene glycol dimethacrylate, tetraethylene glycol diacrylate, pentaerythritol diacrylate, pentaerythritol triacrylate, pentaerythritol tetraacrylate, dipentaerythritol diacrylate, dipentaerythritol triacrylate, dipentaerythritol tetraacrylate, dipentaerythritol pentaacrylate, dipentaerythritol hexaacrylate, tripentaerythritol octaacrylate, pentaerythritol dimethacrylate, pentaerythritol trimethacrylate, dipentaerythritol dimethacrylate, dipentaerythritol tetra-methacrylate, tripentaerythritol octamethacrylate, pentaerythritol diitaconate, dipentaerythritol trisitaconate, dipentaerythritol pentaitaconate, dipentaerythritol hexaitaco-nate, ethylene glycol diacrylate, 1,3-butanediol diacrylate, 1,3-butanediol dimethacrylate, 1,4-butanediol diitaconate, sorbitol triacrylate, sorbitol tetraacrylate, pentaerythritol-modified triacrylate, sorbitol tetramethacrylate, sorbitol pentaacrylate, sorbitol hexaacrylate, oligoester acrylates and methacrylates, glycerol di- and tri-acrylate, 1,4-cyclohexane diacrylate, bisacrylates and bismethacrylates of polyethylene glycol having a molecular weight of from 200 to 1500 and mixtures thereof.
Suitable polymerizable, ethylenically unsaturated compounds are also the amides of identical or different unsaturated carboxylic acids and aromatic, cycloaliphatic and aliphatic polyamines having preferably from 2 to 6, especially from 2 to 4, amino groups. Examples of such polyamines are ethylenediamine, 1,2- or 1,3-propylenediamine, 1,2-, 1,3- or 1,4-butylenediamine, 1,5-pentylenediamine, 1,6-hexylenediamine, octylenediamine, dodecylenediamine, 1,4-diaminocyclohexane, isophoronediamine, phenylenediamine, bisphenylenediamine, di-β-aminoethyl ether, diethylenetri-amine, triethylenetetraamine and di(β-aminoethoxy)- or di(β-aminopropoxy)ethane. Further suitable polyamines are polymers and copolymers which may have additional amino groups in the side chain and oligoamides having amino terminal groups. Examples of such unsaturated amides are: methylene bisacrylamide, 1,6-hexamethylene bisacrylamide, diethylenetriamine trismethacrylamide, bis(methacrylamidopropoxy)ethane, β-methacrylamidoethyl methacrylate and N-[(β-hydroxyethoxy)ethyl]-acrylamide.
Suitable unsaturated polyesters and polyamides are derived, for example, from maleic acid and diols or diamines. The maleic acid may have been partially replaced by other dicarboxylic acids. They can be used together with ethylenically unsaturated comonomers, for example styrene. The polyesters and polyamides can also be derived from dicarboxylic acids and ethylenically unsaturated diols or diamines, especially from those having longer chains of, for example, from 6 to 20 carbon atoms. Examples of polyurethanes are those composed of saturated diisocyanates and unsaturated diols, or unsaturated diisocyanates and saturated diols.
Polybutadiene and polyisoprene and copolymers thereof are known. Suitable comonomers include, for example, olefins, such as ethylene, propene, butene, hexene, (meth)acrylates, acrylonitrile, styrene and vinyl chloride. Polymers having (meth)acrylate groups in the side chain are also known. They may be, for example, reaction products of novolak-based epoxy resins with (meth)acrylic acid; homo- or co-polymers of vinyl alcohol or hydroxyalkyl derivatives thereof that have been esterified with (meth)acrylic acid; or homo- and co-polymers of (meth)acrylates that have been esterified with hydroxyalkyl (meth)acrylates.

Suitable polymerizable, ethylenically unsaturated compounds may also comprise monomeric and/or oligomeric compounds having ethylenically unsaturated bonds (prepolymers) that additionally contain at least one or more OH, NH₂, COOH, epoxy or NCO groups. Of particular interest are, for example, unsaturated acrylates having reactive functional groups. The reactive functional group may be selected, for example, from a hydroxy, thiol, isocyanate, epoxy, anhydride, carboxy, amino or blocked amino group. Examples of OH-group-containing unsaturated acrylates are hydroxyethyl and hydroxybutyl acrylates and also glycidyl acrylates.

The unsaturated compounds may also be used in admixture with non-photopolymerizable film-forming components. These may be, for example, polymers that can be dried physically or solutions thereof in organic solvents, such as nitrocellulose or cellulose acetobutyrate. They may alternatively be chemically or thermally curable resins, such as polyisocyanates, polyepoxides or melamine resins. The concomitant use of thermally curable resins is important for use in so-called hybrid systems which are both photopolymerized and thermally crosslinked.

The polymerizable, ethylenically unsaturated compounds are present in the composition as single components or in any desired mixtures. Preference is given to mixtures of polyol(meth)acrylates.

In a specific embodiment of the invention the polymerizable, ethylenically unsaturated compounds may, for example, be a composition of ethylenically unsaturated compounds comprising
b₁) compounds having one or more free-radically polymerizable double bonds that additionally contain at least one further functional group that is reactive in terms of an addition and/or condensation reaction (examples are given above);
b₂) compounds having one or more free-radically polymerizable double bonds that additionally contain at least one further functional group that is reactive in terms of an addition and/or condensation reaction, the additional reactive functional group being complementary or reactive towards the additional reactive functional groups of component b₁);
b₃) optionally at least one monomeric, oligomeric and/or polymeric compound having at least one functional group that is reactive in terms of an addition and/or condensation reaction towards the functional groups of component b₁) or component b₂) that are present in addition to the free-radically polymerizable double bonds.
Component b₂) in each case carries the groups complementary or reactive towards component b₁). Different types of functional groups may also be present in a component. Component b₃) provides a further component that contains functional groups that are reactive in terms of an addition and/or condensation reaction and that are able to react with the functional groups of b₁) or b₂) that are present in addition to the free-radically polymerizable double bonds. Component b₃) contains no free-radically polymerizable double bonds.
Examples of such combinations b₁), b₂), b₃) can be found in WO 99/55785.
Examples of suitable reactive functional groups are selected, for example, from hydroxy, isocyanate, epoxy, anhydride, carboxy and blocked amino groups. Examples have been described above.

### Additional Components

The curable composition according to the process of the present invention may comprise the following additional components:
c) thermally crosslinkable compound; and/or
d) further additives; and/or
e) additional photoinitiators.

If the composition does not contain any thermally crosslinkable compound as definded under c) it is necessary, in order to obtain a dual curable system, that the polymerizable, ethylenically unsaturated compounds is a mixture of monomeric and/or oligomeric compounds having ethylenically unsaturated bonds (prepolymers) that additionally contain at least one or more OH, SH, NH₂, COOH, epoxy or NCO groups.

According to a preferred embodiment of the invention at least one thermally crosslinkable compound which may contain additional ethylenically unsaturated functionalities is present as component c) in the curable composition.

A preferred embodiment of the invention relates to a process which is characterized in that the composition comprises as further component
c) at least one thermally crosslinkable compound which may contain additional ethylenically unsaturated functionalities;
and the curing is carried out by irradiation with electromagnetic radiation of a wavelength from about 200 nm up to the IR domain combined with prior, simultaneous or subsequent thermal treatment.

Constituents of component c) are, for example, thermally curable lacquer or coating system constituents customary in the art. Component c) may consist of individual or a composition of different constituents.
Component c) is, for example, generally a film-forming binder based on a thermoplastic or thermocurable resin, predominantly on a thermocurable resin. Examples thereof are alkyd, acrylic, polyester, phenol, melamine, epoxy and polyurethane resins and mixtures thereof. Examples thereof are described, for example, in Ullmann's Encyclopedia of Industrial Chemistry, 5th Ed., Vol. A18, Paints and Coatings, pp. 368-426, VCH, DE-Weinheim 1991.
Component c) may also be a cold-curable or hot-curable binder, with the optional addition of a curing catalyst. Suitable catalysts that accelerate the full cure of the binder are described, for example, in Ullmann's Encyclopedia of Industrial Chemistry, Vol. A18, recited above, on page 469.
The amount of binder may be, for example, from 5 to 95% by weight, preferably from 10 to 90% by weight and especially from 40 to 90% by weight, based on the total amount of solids present in the composition. The binder will be selected according to the field of use and the properties required therefor, such as developability in aqueous and organic solvent systems, adhesion to substrates and sensitivity to oxygen.
Suitable binders are, for example, polymers having a molecular weight of approximately from 5000 to 2 000 000, preferably from 10 000 to 1 000 000.
Examples of component c) are, for example, oligomers and/or polymers derived from α,β-unsaturated acids and derivatives thereof, for example polyacrylates and polymethacrylates, polymethyl methacrylates impact-resistant-modified with butyl acrylate, polyacrylamides and polyacrylonitriles. Further examples of component c) are urethanes, polyurethanes derived from either
- polyethers, polyesters and polyacrylates having free hydroxyl groups; or
- aliphatic or aromatic polyisocyanates, and pre-products thereof.
   Component c), accordingly, also includes, for example, crosslinkable acrylic resins derived from substituted acrylic acid esters, for example epoxy acrylates, urethane acrylates and polyester acrylates. Alkyd resins, polyester resins and acrylate resins and modifications thereof that are crosslinked with melamine resins, urea resins, isocyanates, isocyanurates, polyisocyanates, polyisocyanurates and epoxy resins, may also be a constituent of component c).
   Examples of specific binders suitable as component c) are:
   1) surface-coatings based on cold- or hot-crosslinkable alkyd, acrylate, polyester, epoxy or melamine resins or mixtures of such resins, optionally with the addition of a curing catalyst;
   2) two-component polyurethane surface-coatings based on hydroxyl-group-containing acrylate, polyester or polyether resins and aliphatic or aromatic isocyanates, isocyanurates or polyisocyanates;
   3) one-component polyurethane surface-coatings based on blocked isocyanates, isocyanurates or polyisocyanates, which are de-blocked during stoving; it is also possible to add melamine resins as appropriate;
   4) one-component polyurethane surface-coatings based on aliphatic or aromatic urethanes or polyurethanes and hydroxyl-group-containing acrylate, polyester or polyether resins;
   5) one-component polyurethane surface-coatings based on aliphatic or aromatic urethane acrylates or polyurethane acrylates having free amine groups in the urethane structure and melamine resins or polyether resins, optionally with the addition of a curing catalyst;
   6) two-component surface-coatings based on (poly)ketimines and aliphatic or aromatic isocyanates, isocyanurates or polyisocyanates;
   7) two-component surface-coatings based on (poly)ketimines and an unsaturated acrylate resin or a polyacetoacetate resin or a methacrylamidoglycolate methyl ester;
   8) two-component surface-coatings based on carboxyl- or amino-group-containing polyacrylates and polyepoxides;
   9) two-component surface-coatings based on anhydride-group-containing acrylate resins and a polyhydroxy or polyamino component;
   10) two-component surface-coatings based on acrylate-containing anhydrides and polyepoxides;
   11) two-component surface-coatings based on (poly)oxazolines and anhydride-group-containing acrylate resins or unsaturated acrylate resins or aliphatic or aromatic isocyanates, isocyanurates or polyisocyanates;
   12) two-component surface-coatings based on unsaturated polyacrylates and polymalonates;
   13) thermoplastic polyacrylate surface-coatings based on thermoplastic acrylate resins or extrinsically crosslinking acrylate resins in combination with etherified melamine resins;
   14) surface-coating systems based on urethane (meth)acrylate having (meth)acryloyl groups and free isocyanate groups and on one or more compounds that react with isocyanates, for example free or esterified polyols. Such systems have been published, for example, in EP-A-928800.
      A preferred group of binders consists of homo- and co-polymers of acrylates and methacrylates, for example copolymers of methyl methacrylate/ethyl acrylate/methacrylic acid, poly(methacrylic acid alkyl esters), poly(acrylic acid alkyl esters); cellulose esters and ethers, such as cellulose acetate, cellulose acetate butyrate, methyl cellulose, ethyl cellulose; polyvinyl butyral, polyvinylformal, cyclized rubber, polyethers, such as polyethylene oxide, polypropylene oxide, polytetrahydrofuran; polystyrene, polycarbonate, polyurethane, chlorinated polyolefins, polyvinyl chloride, copolymers of vinyl chloride/vinylidene chloride, copolymers of vinylidene chloride with acrylonitrile, methyl methacrylate and vinyl acetate, polyvinyl acetate, copoly(ethylene/vinyl acetate), polymers, such as polycaprolactam and poly(hexamethylene-adipamide), polyesters, such as poly(ethylene glycol terephthalate) and poly(hexamethylene glycol succinate).

Blocked isocyanates may also be used in component c). These are described, for example, in Organischer Metallschutz: Entwicklung und Anwendung von Beschichtungsstoffen, Vincentz Verlag, DE-Hannover (1993), pages 159-160*.* These are compounds in which the highly reactive NCO group is "blocked" by reaction with specific radicals, for example primary alcohols, phenol, acetoacetic ester, ε-caprolactam, phthalimide, imidazole, oxime or amine. The blocked isocyanate is stable in liquid systems and also in the presence of hydroxy groups. Upon heating, the blocking agent is removed again and the NCO group is freed.
1-Component (1C) and 2-component systems (2C) may be used as component c). Examples of such systems are described in Ullmann's Encyclopedia of Industrial Chemistry, Vol. A18, loc. cit., pages 404-407.
It is possible to optimize the composition by specially adapting the composition, for example by varying the binder/crosslinking agent ratio. Such measures will be known to the person skilled in the art of surface-coating technology.
In the curing process according to the invention, component c) is preferably a mixture based on acrylate/melamine (and melamine derivatives), 2-component polyurethane, 1-component polyurethane, 2-component epoxy/carboxy or 1-component epoxy/carboxy. Mixtures of those systems are also possible, for example the addition of melamine (or derivatives thereof) to 1-component polyurethanes.
Component c) is preferably a binder based on a polyacrylate with melamine or on a melamine derivative. Preference is also given to a system based on a polyacrylate and/or polyester polyol with an unblocked polyisocyanate or polyisocyanurate.
Component c) may also comprise monomeric and/or oligomeric compounds having ethylenically unsaturated bonds (prepolymers) that additionally contain at least one or more OH, NH₂, COOH, epoxy or NCO groups (= c₁) that are capable of a reaction with the binder and/or the crosslinking agent constituent of component c). After application and thermal curing, the ethylenically unsaturated bonds are converted to a crosslinked, high molecular weight form by UV-radiation. Examples of such components c) are described, for example, in the above-mentioned reference book, Ullmann's Encyclopedia of Industrial Chemistry, Vol. A18, recited above, on pages 451-453, or in S. Urano, K. Aoki, N. Tsuboniva and R. Mizuguchi, Progress in Organic Coatings, 20 (1992), 471-486*,* or H. Terashima and O. Isozaki, JOCCA 1992 (6), 222.
Component c₁) may, for example, be an OH-group-containing unsaturated acrylate, for example hydroxyethyl or hydroxybutyl acrylate or a glycidyl acrylate. Component c₁) may be of any desired structure (for example it may contain units of polyester, polyacrylate, polyether, etc..), provided that it contains an ethylenically unsaturated double bond and additionally free OH, COOH, NH₂, epoxy or NCO groups.
Component c₁) may, for example, also be obtained by reacting an epoxy-functional oligomer with acrylic acid or methacrylic acid. A typical example of an OH-functional oligomer having vinylic double bonds is 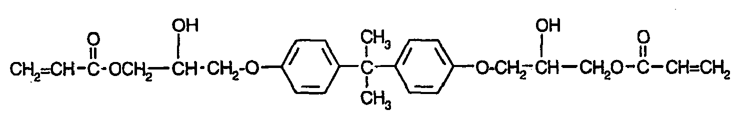 obtained by reaction of CH₂=CHCOOH with

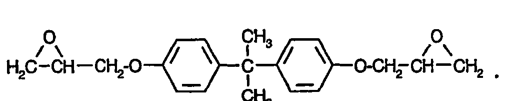

Another possible method of preparing component c₁) is, for example, the reaction of an oligomer that contains only one epoxy group and has a free OH group at another position in the molecule.
A preferred embodiment of the invention relates to a process which is characterized in that the thermally crosslinkable compound c) is a binder based on a composition of a polyacrylate with melamine or a melamine derivative, or a composition based on a polyacrylate polyol and a polyester polyol with an unblocked polyisocyanate or polyisocyanurate or a polyester polyol with an unblocked polyisocyanate or polyisocyanurate.
The curable composition according to the process of the present invention may comprise various additives d) in addition to the components recited above. Examples thereof are thermal inhibitors, the purpose of which is to prevent premature polymerization, for example 2,2,6,6-tetramethyl-4-hydroxy-piperidin-1-oxyl (4-hydroxy-TEMPO) and derivatives thereof, e.g. bis(2,2,6,6-tetramethylpiperidin-1-oxyl-4-yl)-decanedioate or polyalkyl-piperidine-N-oxyl radicals, 3-aryl-benzofuran-2-one and derivatives thereof, e.g. 5,7-di-tert-butyl-3-phenyl-3H-benzofuran-2-one, hydroquinone, hydroquinone derivatives, p-methoxyphenol, β-naphthol and sterically hindered phenols, for example 2,6-di(tert-butyl)-p-cresol. In order to increase stability to dark storage, it is possible, for example, to use copper compounds, such as copper naphthenate, stearate or octanoate, phosphorus compounds, for example triphenylphosphine, tributylphosphine, triethyl phosphite, triphenyl phosphite or tribenzyl phosphite, quaternary ammonium compounds, for example tetramethylammonium chloride or trimethylbenzylammonium chloride, or hydroxylamine derivatives, for example N-diethylhydroxylamine. In order to exclude atmospheric oxygen during polymerization, it is possible to add paraffin or similar wax-like substances that, being insufficiently soluble in the polymer, migrate to the surface at the beginning of the polymerization and form a transparent surface layer which prevents the ingress of air. Equally possible is the application of an oxygen-impermeable layer. UV-Absorbers, for example of the hydroxyphenyl-benzotriazole, hydroxyphenyl-benzophenone, oxalic acid amide or hydroxyphenyl-s-triazine type, may be added as light stabilizers. Individual compounds or mixtures of those compounds may be used with or without the use of sterically hindered amines (HALS).

Examples of such UV-absorbers and light stabilizers are
1. 2-(2'-Hydroxyphenyl)benzotriazoles, for example 2-(2'-hydroxy-5'-methylphenyl)benzotriazole, 2-(3',5'-di-tert-butyl-2'-hydroxyphenyl)benzotriazole, 2-(5'-tert-butyl-2'-hydroxy-phenyl)benzotriazole, 2-(2'-hydroxy-5'-(1,1,3,3-tetramethylbutyl)phenyl)benzotriazole, 2-(3',5'-di-tert-butyl-2'-hydroxyphenyl)-5-chloro-benzotriazole, 2-(3'-tert-butyl-2'-hydroxy-5'-methylphenyl)-5-chloro-benzotriazole, 2-(3'-sec-butyl-5'-tert-butyl-2'-hydroxyphenyl)benzo-triazole, 2-(2'-hydroxy-4'-octyloxyphenyl)benzotriazole, 2-(3',5'-di-tert-amyl-2'-hydroxyphenyl)benzotriazole, 2-(3',5'-bis-(α,α-dimethylbenzyl)-2'-hydroxyphenyl)benzotriazole, 2-(3'-tert-butyl-2'-hydroxy-5'-(2-octyloxycarbonylethyl)phenyl)-5-chloro-benzotriazole, 2-(3'-tert-butyl-5'-[2-(2-ethylhexyloxy)-carbonylethyl]-2'-hydroxyphenyl)-5-chlorobenzotriazole, 2-(3'-tert-butyl-2'-hydroxy-5'-(2-methoxycarbonylethyl)phenyl)-5-chlorobenzotriazole, 2-(3'-tert-butyl-2'-hydroxy-5'-(2-methoxycarbonylethyl)phenyl)benzotriazole, 2-(3'-tert-butyl-2'-hydroxy-5'-(2-octyloxycarbonyethyl)phenyl)benzotriazole, 2-(3'_tert-butyl-5'-[2-(2-ethylhexyl-oxy)carbonylethyl]-2'-hydroxyphenyl)benzotriazole, 2-(3'-dodecyl-2'-hydroxy-5'-methyl-phenyl)benzotriazole, 2-(3'-tert-butyl-2'-hydroxy-5'-(2-iso-octyloxycarbonylethyl)-phenyl-benzotriazole, 2,2'-methylene-bis[4-(1,1,3,3-tetramethylbutyl)-6-benzotriazol-2-yl-phenol]; the transesterification product of 2-[3'-tert-butyl-5'-(2-methoxycarbonylethyl)-2'-hydroxyphenyl]-2H-benzotriazole with polyethylene glycol 300; [R-CH₂CH₂-COO-CH₂CH₂]₂- where R = 3'-tert-butyl-4'-hydroxy-5'-2H-benzotriazol-2-ylphenyl, 2-[2'-hydroxy-3'-(a,a-dimethylbenzyl)-5'-(1,1,3,3-tetramethylbutyl)-phenyl]benzotriazole; 2-[2'-hydroxy-3'-(1,1,3,3-tetramethylbutyl)-5'-(α,α-dimethylbenzyl)-phenyl]benzotriazole;
2. 2-Hydroxybenzophenones, for example the 4-hydroxy, 4-methoxy, 4-octyloxy, 4-decyloxy, 4-dodecyloxy, 4-benzyloxy, 4,2',4'-trihydroxy and 2'-hydroxy-4,4'-dimethoxy derivatives;
3. Esters of substituted and unsubstituted benzoic acids, as for example 4-tert-butyl-phenyl salicylate, phenyl salicylate, octylphenyl salicylate, dibenzoyl resorcinol, bis(4-tert-butylbenzoyl) resorcinol, benzoyl resorcinol, 2,4-di-tert-butylphenyl 3,5-di-tert-butyl-4-hydroxybenzoate, hexadecyl 3,5-di-tert-butyl-4-hydroxybenzoate, octadecyl 3,5-di-tert-butyl-4-hydroxybenzoate, 2-methyl-4,6-di-tert-butylphenyl 3,5-di-tert-butyl-4-hydroxybenzoate;
4. Acrylates, for example ethyl α-cyano-β,β-diphenylacrylate, isooctyl α-cyano-β,β-diphenyl-acrylate, methyl α-methoxycarbonylcinnamate, methyl α-cyano-β-methyl-p-methoxy-cin-namate, butyl α-cyano-β-methyl-p-methoxy-cinnamate, methyl α-methoxycarbonyl-p-methoxycinnamate and N-(β-methoxycarbonyl-β-cyanovinyl)-2-methylindoline;
5. Sterically hindered amines, for example bis(2,2,6,6-tetramethyl-4-piperidyl)sebacate, bis-(2,2,6,6-tetramethyl-4-piperidyl)succinate, bis(1,2,2,6,6-pentamethyl-4-piperidyl)-sebacate, bis-(1-octyloxy-2,2,6,6-tetramethyl-4-piperidyl)sebacate, bis(1,2,2,6,6-penta-methyl-4-piperidyl)-n-butyl-3,5-di-tert-butyl-4-hydroxybenzylmalonate, the condensate of 1-(2-hydroxyethyl)-2,2,6,6-tetramethyl-4-hydroxypiperidine and succinic acid, linear or cyclic condensates of N,N'-bis(2,2,6,6-tetramethyl-4-piperidyl)hexamethylenediamine and 4-tert-octylamino-2,6-dichloro-1,3,5-triazine, tris(2,2,6,6-tetramethyl-4-piperidyl)nitrilotriacetate, tetrakis(2,2,6,6-tetramethyl-4-piperidyl)-1,2,3,4-butane-tetracarboxylate, 1,1'-(1,2-ethanediyl)-bis(3,3,5,5-tetramethylpiperazinone), 4-benzoyl-2,2,6,6-tetramethylpiperidine, 4-stearyl-oxy-2,2,6,6-tetramethylpiperidine, bis(1,2,2,6,6-pentamethylpiperidyl)-2-n-butyl-2-(2-hydroxy-3,5-di-tert-butylbenzyl)malonate, 3-n-octyl-7,7,9,9-tetramethyl-1,3,8-triaza-spiro-[4.5]decane-2,4-dione, bis(1-octyloxy-2,2,6,6-tetramethylpiperidyl)sebacate, bis(1-octyloxy-2,2,6,6-tetramethylpiperidyl)succinate, linear or cyclic condensates of N,N'-bis-(2,2,6,6-tetramethyl-4-piperidyl)hexamethylenediamine and 4-morpholino-2,6-dichloro-1,3,5-triazine, the condensate of 2-chloro-4,6-bis(4-n-butylamino-2,2,6,6-tetramethylpiperidyl)-1,3,5-triazine and 1,2-bis(3-aminopropylamino)ethane, the condensate of 2-chloro-4,6-di-(4-n-butylamino-1,2,2,6,6-pentamethylpiperidyl)1,3,5-triazine and 1,2-bis(3-aminopropyl-amino)ethane, 8-acetyl-3-dodecyl-7,7,9,9-tetramethyl-1,3,8-triazaspiro[4.5]decane-2,4-dione, 3-dodecyl-1-(2,2,6,6-tetramethyl-4-piperidyl)pyrrolidine-2,5-dione, 3-dodecyl-1-(1,2,2,6,6-pentamethyl-4-piperidyl)pyrrolidine-2,5-dione, a mixture of 4-hexadecyloxy- and 4-stearyloxy-2,2,6,6-tetramethylpiperidine, a condensation product of N,N'-bis(2,2,6,6-tetramethyl-4-piperidyl)hexamethylenediamine and 4-cyclohexylamino-2,6-dichloro-1,3,5-triazine, a condensation product of 1,2-bis(3-aminopropylamino)ethane and 2,4,6-trichloro-1,3,5-triazine as well as 4-butylamino-2,2,6,6-tetramethylpiperidine (CAS Reg. No. [136504-96-6]); N-(2,2,6,6-tetramethyl-4-piperidyl)-n-dodecylsuccinimide, N-(1,2,2,6,6-pentamethyl-4-piperidyl)-n-dodecylsuc-cinimide, 2-undecyl-7,7,9,9-tetramethyl-1-oxa-3,8-diaza-4-oxo-spiro[4,5]decane, a reaction product of 7,7,9,9-tetramethyl-2-cycloundecyl-1-oxa-3,8-diaza-4-oxospiro[4,5]decane and epichlorohydrin, 1,1-bis(1,2,2,6,6-pentamethyl-4-piper-idyloxycarbonyl)-2-(4-methoxyphenyl)ethene, N,N'-bis-formyl-N,N'-bis(2,2,6,6-tetra-methyl-4-piperidyl)hexamethylenediamine, diester of 4-methoxy-methylene-malonic acid with 1,2,2,6,6-pentamethyl-4-hydroxypiperidine, poly[methylpropyl-3-oxy-4-(2,2,6,6-tetramethyl-4-piperidyl)]siloxane, reaction product of maleic acid anhydride-a-olefin-copolymer with 2,2,6,6-tetramethyl-4-aminopiperidine or 1,2,2,6,6-pentamethyl-4-aminopiperidine;
6. Oxamides, for example 4,4'-dioctyloxyoxanilide, 2,2'-diethoxyoxanilide, 2,2'-dioctyloxy-5,5'-di-tert-butoxanilide, 2,2'-didodecyloxy-5,5'-di-tert-butoxanilide, 2-ethoxy-2'-ethyloxanilide, N,N'-bis(3-dimethylaminopropyl)oxamide, 2-ethoxy-5-tert-butyl-2'-ethoxanilide and its mixture with 2-ethoxy-2'-ethyl-5,4'-di-tert-butoxanilide, mixtures of o- and p-methoxydisubstituted oxanilides and mixtures of o- and p-ethoxy-disubstituted oxanilides;
7. 2-(2-Hydroxyphenyl)-1,3,5-triazines, for example 2,4,6-tris(2-hydroxy-4-octyloxyphenyl)-1,3,5-triazine, 2-(2-hydroxy-4-octyloxyphenyl)-4,6-bis(2,4-dimethylphenyl)-1,3,5-triazine, 2-(2,4-dihydroxyphenyl)-4,6-bis(2,4-dimethylphenyl)-1,3,5-triazine, 2,4-bis(2-hydroxy-4-propyloxyphenyl)-6-(2,4-dimethylphenyl)-1,3,5-triazine, 2-(2-hydroxy-4-octyloxyphenyl)-4,6-bis(4-methylphenyl)-1,3,5-triazine, 2-(2-hydroxy-4-dodecyloxyphenyl)-4,6-bis(2,4-dimethylphenyl)-1,3,5-triazine, 2-(2-hydroxy-4-tridecyloxyphenyl)-4,6-bis(2,4-dimethylphenyl)-1,3,5-triazine, 2-[2-hydroxy-4-(2-hydroxy-3-butyloxy-propoxy)phenyl]-4,6-bis(2,4-dimethyl)-1,3,5-triazine, 2-[2-hydroxy-4-(2-hydroxy-3-octyloxy-propyloxy)phenyl]-4,6-bis(2,4-dimethyl)-1,3,5-triazine, 2-[4-(dodecyloxy/tridecyloxy-2-hydroxypropoxy)-2-hydroxyphenyl]-4,6-bis(2,4-dimethyl-phenyl)1,3,5-triazine, 2-[2-hydroxy-4-(2-hydroxy-3-dodecyloxy-propoxy)phenyl]-4,6-bis(2,4-dimethylphenyl)-1,3,5-triazine, 2-(2-hydroxy-4-hexyloxy)phenyl-4,6-diphenyl-1,3,5-triazine, 2-(2-hydroxy-4-methoxyphenyl)-4,6-diphenyl-1,3,5-triazine, 2,4,6-tris[2-hydroxy-4-(3-butoxy-2-hydroxy-propoxy)phenyl]-1,3,5-triazine, 2-(2-hydroxyphenyl)-4-(4-methoxyphenyl)-6-phenyl-1,3,5-triazine, 2-{2-hydroxy-4-[3-(2-ethylhexyl-1-oxy)-2-hydroxypropyloxy]phenyl}-4,6-bis(2,4-dimethylphenyl)-1,3,5-triazine;
8. Phosphites and phosphonites, for example triphenyl phosphite, diphenyl alkyl phosphites, phenyl dialkyl phosphites, tris(nonylphenyl) phosphite, trilauryl phosphite, trioctadecyl phosphite, distearyl pentaerythritol diphosphite, tris(2,4-di-tert-butylphenyl) phosphite, diisodecyl pentaerythritol diphosphite, bis(2,4-di-tert-butylphenyl) pentaerythritol diphosphite, bis(2,6-di-tert-butyl-4-methylphenyl)-pentaerythritol diphosphite, diisodecyloxypentaerythritol diphosphite, bis(2,4-di-tert-butyl-6-methylphenyl)pentaerythritol diphosphite, bis(2,4,6-tris(tert-butylphenyl)pentaerythritol diphosphite, tristearyl sorbitol triphosphite, tetrakis(2,4-di-tert-butylphenyl) 4,4'-biphenylene diphosphonite, 6-isooctyloxy-2,4,8,10-tetra-tert-butyl-12H-dibenz[d,g]-1,3,2-dioxaphosphocine, bis(2,4-di-tert-butyl-6-methylphenyl) methyl phosphite, bis(2,4-di-tert-butyl-6-methylphenyl) ethyl phosphite, 6-fluoro-2,4,8,10-tetra-tert-butyl-12-methyl-dibenz[d,g]-1,3,2-dioxaphosphocine, 2,2',2"-nitrilo[triethyltris(3,3',5,5'-tetra-tert-butyl-1,1'-biphenyl-2,2'-diyl)phosphite], 2-ethylhexyl-(3,3',5,5'-tetra-tert-butyl-1,1'-biphenyl-2,2'-diyl)phosphite, 5-butyl-5-ethyl-2-(2,4,6-tri-tert-butylphenoxy)-1,3,2-dioxaphosphirane.

In order to accelerate the photopolymerization step it is possible to add as further additives d) amines, for example triethanolamine, N-methyl-diethanolamine, p-dimethylaminobenzoic acid ethyl ester or Michler's ketone. The action of the amines can be enhanced by the addition of aromatic ketones of the benzophenone type. Amines that can be used as oxygen capture agents are, for example, substituted N,N-dialkylanilines, as described in EP-A-0 339 841. Further accelerators, co-initiators and auto-oxidizers are thiols, thio ethers, disulfides and phosphines, as described, for example, in EP-A-0 438 123 and GB-A-2,180,358.

It is also possible to add to the compositions chain-transfer reagents customary in the art, examples of which are mercaptans, amines and benzothiazole.
Photopolymerization can also be accelerated by adding as further additives d) photosensitizers that shift or broaden the spectral sensitivity. These include especially aromatic carbonyl compounds, such as benzophenone and thioxanthone, especially also isopropylthioxanthone, anthraquinone and 3-acylcoumarin derivatives, terphenyls, styryl ketones, and 3-(aroylmethylene)-thiazolines, camphorquinone, and also eosin, rhodamine and erythrosine dyes.
The above-mentioned amines may, for example, also be regarded as photosensitizers. The curing process, especially in the case of pigmented compositions, for example compositions pigmented with titanium dioxide, may also be assisted by the use of an additional additive d) that is a component that forms free radicals under thermal conditions, for example an azo compound, such as 2,2'-azobis(4-methoxy-2,4-dimethylvaleronitrile), a triazene, diazosulfide, pentaazadiene or a peroxo compound, such as a hydrogen peroxide or peroxycarbonate, for example tert-butyl hydroperoxide, as described, for example, in EP-A-0 245 639.
The compositions may also comprise as further additives d), for example, a photoreducible dye, for example xanthene, benzoxanthene, benzothioxanthene, thiazine, pyronine, porphyrin or acridine dyes, and/or a radiation-cleavable trihalomethyl compound. Similar compositions are described, for example, in EP-A-0 445 624.
Further customary additives d) are - depending upon the intended use - optical brighteners, fillers, for example kaolin, talcum, barytes, gypsum, chalk or silicate-like fillers, pigments, dyes, wetting agents and flow improvers.

For the curing of thick and pigmented coatings it is suitable to add glass microbeads or pulverized glass fibers, as described, for example, in US-A-5,013,768.
The composition may also comprise colorants and/or white or colored pigments. Depending upon the intended use, both inorganic and organic pigments may be used. Such additives are known to the person skilled in the art; some examples thereof are titanium dioxide pigments, for example of the rutile or anatase type, carbon black, zinc oxide, such as zinc white, iron oxides, such as iron oxide yellow, iron oxide red, chromium yellow, chromium green, nickel titanium yellow, ultramarine blue, cobalt blue, bismuth vanadate, cadmium yellow and cadmium red. Examples of organic pigments are mono- and bis-azo pigments, and metal complexes thereof, phthalocyanine pigments, polycyclic pigments, such as perylene, anthraquinone, thioindigo, quinacridone and triphenylmethane pigments, and diketo-pyrrolopyrrole, isoindolinone, for example tetrachloroisoindolinone, isoindoline, dioxazine, benzimidazolone and quinophthalone pigments. These pigments may be added to the compositions individually or in admixture with other components.
Depending upon the intended use, the pigments are added to the composition in amounts customary in the art, for example in an amount of from 1 to 60% by weight, or from 10 to 30% by weight, based on the total weight.
The composition may, for example, also comprise organic colorants of a wide variety of classes, examples of which are azo dyes, methine dyes, anthraquinone dyes and metal complex dyes. Customary concentrations are, for example, from 0.1 to 20%, especially from 1 to 5%, based on the total weight.
The composition which is subjected to dual cure may also comprise thermal drying or curing catalysts as additional additives d). Examples of thermal drying or curing catalysts are, for example, organic metal compounds, amines and/or phosphines. Organic metal compounds are, for example, metal carboxylates, especially those of the metals Pb, Mn, Hf, Co, Zn, Zr or Cu, or metal chelates, especially those of the metals Hf, Al, Ti or Zr, or organometal compounds, for example organotin compounds. Examples of metal carboxylates are the stearates of Pb, Mn or Zn, the octanoates of Co, Zn or Cu, the naphthenates of Mn and Co or the corresponding linoleates or tallates. Examples of metal chelates are aluminum, titanium or zirconium chelates of acetylacetone, ethyl acetylacetate, salicylaldehyde, salicylaldoxime, o-hydroxyacetophenone or ethyl trifluoroacetylacetate and the alkoxides of these metals. Examples of organotin compounds are dibutyltin oxide, dibutyltin dilaurate and dibutyltin dioctanoate. Examples of amines are especially tertiary amines, for example tributylamine, triethanolamine, N-methyl-diethanolamine, N-dimethylethanolamine, N-ethylmorpholine, N-methylmorpholine or diazabicyclooctane (triethylenediamine) and salts thereof. Further examples are, for example trimethylbenzylammonium chloride. Phosphines, for example triphenylphosphine, can also be used as curing catalysts. Suitable catalysts are also described, for example, in the reference book J. Bielemann, Lackadditive, Wiley-VCH Verlag GmbH, DE-Weinheim, 1998, pages 244-247. Examples thereof are carboxylic acids, such as p-toluenesulfonic acid, dodecylbenzenesulfonic acid, dinonylnaphthalenesulfonic acid and dinonylnaphthalenedisulfonic acid. Latent or blocked sulfonic acids may, for example, also be used, it being possible for the blocking of the acid to be ionic or non-ionic. Such catalysts are used in concentrations customary in the art and known to the person skilled in the art.
The choice of the additives recited above will depend upon the field of use in question and upon the properties desired for that field. The additives d) described above are customary in the art and are accordingly used in amounts customary in the art.
The curable composition according to the process of the present invention may comprise various known photoinitiators e) in addition to the photoinitiators a) and the other components recited above. Examples thereof are mixtures with known photoinitiators, for example mixtures with camphorquinone, benzophenone, benzophenone derivatives, acetophenone, acetophenone derivatives, such as α-hydroxycycloalkylphenylketones or 2-hydroxy-2-methyl-1-phenyl-propanone, dialkoxyacetophenones, α-hydroxy- or α-amino-acetophenones, for example (4-methylthiobenzoyl)-1-methyl-1-morpholino-ethane, (4-morpholino-benzoyl)-1-benzyl-1-dimethylamino-propane, 4-aroyl-1,3-dioxolanes, benzoin alkyl ethers and benzil ketals, for example benzil dimethyl ketal, phenyl glyoxalates and derivatives thereof, dimeric phenyl glyoxalates, peresters, for example benzophenone tetracarboxylic acid peresters, as described, for example, in EP-A-0 126 541, monoacylphosphine oxides, for example (2,4,6-trimethylbenzoyl)-phenyl-phosphine oxide, bisacylphosphine oxides, for example bis(2,6-dimethoxybenzoyl)-(2,4,4-trimethyl-pent-1-yl)phosphine oxide, bis(2,4,6-trimethylbenzoyl)-phenyl-phosphine oxide or bis(2,4,6-trimethylbenzoyl)-(2,4-dipentoxyphenyl)phosphine oxide, trisacylphosphine oxides, halomethyltriazines, for example 2-[2-(4-methoxy-phenyl)-vinyl]-4,6-bis-trichloromethyl-[1,3,5]triazine, 2-(4-methoxy-phenyl)-4,6-bis-trichloromethyl-[1,3,5]triazine, 2-(3,4-dimethoxy-phenyl)-4,6-bis-trichloro-methyl-[1,3,5]triazine, 2-methyl-4,6-bis-trichloromethyl-[1,3,5]triazine, hexaarylbisimidazole/co-initiator systems, for example ortho-chlorohexaphenyl-bisimidazole together with 2-mercaptobenzothiazole, ferrocenium compounds or titanocenes, for example dicyclopentadienyl-bis(2,6-difluoro-3-pyrrolophenyl)titanium or borate photoinitiators.
The photoinitiators listed above may be used in hybrid systems, that is to say in systems that are both free-radically and cationically curable, in addition with further free-radical hardeners cationic photoinitiators, for example benzoyl peroxide. Other suitable peroxides are described in US-A-4,950,581, v. column 19, lines 17-25. Aromatic sulfonium, phosphonium or iodonium salts may also be used, such as the ones described, for example, in US-A-4,950,581, v. column 18, line 60 to column 19, line 10.
The photopolymerizable compositions contain the photoinitiators advantageously in an amount of from 0.05 to 15% by weight, preferably from 0.1 to 5% by weight, based on the composition. The amount of photoinitiator indicated relates to the sum of all the photoinitiators added when mixtures thereof are used, that is to say either to the photoinitiator a) or to the combined photoinitiators a) and e).
The quantity ratio of the components a) to e) in the UV- and thermally-crosslinking compositions is not critical. "Dual-cure" systems are known to the person skilled in the art, who will therefore be familiar with the optimum ratios of the UV- and thermally-crosslinkable components for a particular desired use. For example, compositions may contain components b) and c), for example, in a ratio of from 5 : 95 to 95 : 5, from 20: 80 to 80 : 20 or from 30 : 70 to 70 : 30, for example from 40 : 60 to 60 : 40.
Examples of "dual-cure" systems, that is to say systems comprising both UV-curable and thermally curable components, can be found *inter alia* in US-A-5,922,473, columns 6 to 10. It is also possible to add solvent or water to the compositions used in the process according to the invention. If the compositions are used without solvent, they are, for example, powder coating compositions.
The powder coating compositions may be based on solid resins and monomers containing reactive double bonds, for example maleates, vinyl ethers, acrylates, acrylamides and mixtures thereof. A powder coating composition can be formulated by mixing unsaturated polyester resins with solid acrylamides (for example methylacrylamidoglycolate methyl ester) and a free-radical photoinitiator of the formula I defined above. Powder coating compositions can also be formulated by mixing unsaturated polyester resins with solid acrylates, methacrylates or vinyl ethers and a photoinitiator (or photoinitiator mixture) according to the invention. The powder coating compositions may also comprise binders, as described, for example, in DE -A-4 228 514 and EP-A-0 636 669, such as:
1) an unsaturated resin from the group of (semi)crystalline or amorphous unsaturated polyesters, unsaturated polyacrylates or mixtures thereof with unsaturated polyesters, special preference being given to those derived from maleic acid or fumaric acid;
2) an oligomeric or polymeric crosslinking agent having vinyl ether-, vinyl ester- or (meth)acrylate-functional groups, special preference being given to vinyl ether oligomers, such as divinyl-ether-functionalized urethanes; and
3) the photoinitiator.
   The preparation of the coating is performed by applying the dry or liquid composition, a solution or suspension to the substrate. The choice of the solvent and the concentration are governed chiefly by the nature of the composition and by the coating method. Suitable solvents are known to the person skilled in the art, especially those customary in surface-coating technology. The solvent should be inert, that is to say it should not enter into any chemical reaction with the components and it should be capable of being removed again upon drying after the coating operation. Examples of suitable solvents are ketones, ethers and esters, such as methyl ethyl ketone, isobutyl methyl ketone, cyclopentanone, cyclohexanone, N-methylpyrrolidone, diethylene glycol monoethyl ether, dipropylene glycol diethyl ether dioxane, tetrahydrofuran, 2-methoxyethanol, 2-ethoxyethanol, 1-methoxy-2-propanol, 1,2-dimethoxyethane, ethyl acetate, n-butyl acetate, ethyl 3-ethoxy-propionate, aliphatic hydrocarbons, such as hexane, octane, decane; and petroleum solvents, for example petroleum ether or aromatic hydrocarbons, for example toluene or xylene.
   The composition is applied uniformly to a substrate by known coating methods, for example by spin-coating, immersion, knife coating, curtain pouring, brush application or spraying, especially by electrostatic spraying and reverse-roll coating, and by electrophoretic deposition. It is also possible to apply the photosensitive layer to a temporary flexible support and then coat the final substrate by transferring the layer by lamination.
   The amount applied (layer thickness) and the type of substrate (layer support) are dependent upon the desired field of use. The dry layer thickness range generally includes values from about 0.1 µ to more than 100 µ, preferably from 0.02 to 2 cm.
   A further application for dual cure is in metal coating, for example in the surface-coating of metal sheets and tubes, cans or bottle closures, and curing on plastics coatings, for example of PVC-based floor or wall coverings. Examples of curing of paper coatings are the application of a colorless surface-coating to labels, record sleeves or book covers.
   The photosensitivity of the compositions used in the process according to the invention generally ranges from about 200 nm to about 600 nm (UV field). Suitable radiation is present, for example, in sunlight or light from artificial light sources. Accordingly, a large number of widely varying types of light sources may be used. Point sources and also planiform radiators (lamp carpets) are suitable. Examples thereof include: carbon arc lamps, xenon arc lamps, medium-, high- and low-pressure mercury arc lamps, doped where appropriate with metal halides (metal halide lamps), microwave-excited metal vapor lamps, excimer lamps, superactinic fluorescent tubes, fluorescent lamps, argon incandescent lamps, flashlamps, photographic flood lights, light-emitting diodes (LED), electron beams and X-rays. The distance between the lamp and the substrate to be irradiated can vary according to the intended use and upon the type and strength of the lamp, and may be, for example, from 2 cm to 150 cm. Laser light sources, for example excimer lasers, such as Krypton F lasers for irradiation at 248 nm, are especially suitable. Lasers in the visible range can also be used.
   As mentioned before, in the process according to the invention, curing can be effected by irradiation with electromagnetic radiation combined with thermal curing before, during or after the irradiation.
   The thermal curing is carried out according to methods known to the person skilled in the art. Curing is generally carried out in an oven, for example a circulating-air oven, on a hotplate or by irradiation with IR lamps. Curing at room temperature without aids is also possible, depending upon the binder system used. The curing temperatures are generally from room temperature to 200°C, for example from 25°C to 150°C or from 50°C to 150°C or from 80°C to 200°C. In the case of powder coating compositions or "coil coat" surface-coatings, the curing temperatures may even be higher, for example up to 350°C.
   The compositions defined above used in the process according to the invention are suitable, for example, as coating materials for all kinds of substrates, for example wood, textiles, paper, ceramics, glass, plastics, such as polyesters, polyethylene terephthalate, polyolefins or cellulose acetate, especially in the form of films on metals, such as Al, Cu, Ni, Fe, Zn, Mg, Co or alloys thereof and semiconductors, such as GaAs or Si to which a protective layer is to be applied or an image is to be applied by image-wise exposure.
   The coated substrate that is coated on at least one surface with the composition defined above is another embodiment of the present invention.
   The compositions defined above used in the process according to the invention are suitable for a variety of specific purposes, for example as printing inks, as clear lacquers, as white surface-coating compositions, as color-pigmented surface-coating compositions, for example for wood or metal, as powder coating compositions, as paint, *inter alia*, for paper, wood, metal or plastics, as daylight-curable paint for marking structures and roads, for photographic reproduction processes, for holographic recording materials, for image recording processes or for the production of printing plates that are to be developed with organic solvents or using aqueous/alkaline media, in the production of masks for screen-printing, as dental filling compounds, as adhesives, as pressure-sensitive adhesives, as laminating resins, as etch resists or permanent resists, liquid films and dry films, as photostructurable dielectrics, and as solder masks for electronic circuits, as resists in the manufacture of color filters for any type of screen or for producing structures in the manufacturing process of plasma displays and electroluminescent displays, in the manufacture of optical switches, optical gratings (interference gratings), in the manufacture of three-dimensional articles by means of bulk curing (UV-curing in transparent moulds) or using the stereolithography process, as described, for example, in US-A-4,575,330, in the manufacture of composites (e.g. styrene polyesters that may optionally include glass fibers and/or other fibers and other adjuvants) and other thick-layered compositions, in the coating or sealing of electronic components or as coatings for optical fibers. The compositions are also suitable for the manufacture of optical lenses, for example contact lenses or Fresnel lenses, and also in the manufacture of medical apparatus, aids or implants. The compositions can also be used in the manufacture of gels having thermotropic properties, as described, for example, in DE-A-19 700 064 and EP-A-0 678 534.
   It is known that the industrial applicability of the photoinitiators used in the process for the preparation of scratch resistant durable coatings depends on the phase properties of the photoinitiator molecules when applied with the composition to the substrate. To obtain scratch resistant durable coatings photoiniator molecules are preferably concentrated at the surface of the coating to be cured. This is achieved by orienting non-polar groups of a surface-active photoinitiator towards the surface of coatings. The polymerization initiators (I) have the beneficial effect that their concentration at the surface of coatings is increased.

The present invention also relates to a process for concentrating a surface-active photoinitiator in the surface of coatings by dual cure which is **characterized in that** a composition comprising
a) at least one polymerization initiator (I), wherein R₁- R₇ and X are as defined above; and
b) polymerizable, ethylenically unsaturated compounds; and, optionally, as further components; a thermally crosslinkable compound; and/or further additives; and/or additional photoinitiators;
   is applied to a support and cured by irradiation with electromagnetic radiation of a wavelength from about 200 nm up to the IR domain combined with prior, simultaneous or subsequent thermal treatment.

The photoinitiators (I) provided with non-polar groups can also serve as flow improves since they are oriented towards the surface and influence the surface properties by the presence of non-polar groups. It is also possible to add further flow improvers customary in the art. Examples thereof are siloxane compounds and fluorohydrocarbon compounds, which are commercially available.
The invention relates also to the use of compounds of formula I as flow improvers, optionally in combination with other customary flow improvers.
Flow is defined, according to ***DIN 55945***, as "the ability, to a greater or lesser degree, of a still liquid paint to level out, by itself, any unevenness arising during its application" cf. J. Bielemann, Lackadditive, VCH Weinheim 1998, Chapter 6. The flow of a coating composition is highly dependent upon its flow behavior and its surface tension. The term "flow improver" is used to denote a substance that, by lowering the viscosity and/or the surface tension, enables wet coatings to become evenly flowing films. In the case of powder coating compositions, flow improvers also lower the melt viscosity and the glass transition temperature, and they also act as de-gassing agents. The use of flow improvers eliminates flow and surface faults that impair the overall appearance of the coating. Flow and surface faults include, *inter alia*, orange peel effect, structure formation, scratching, fisheye formation, sensitivity to draught, substrate wetting problems, brush application marks, run formation, stippling, pinholes, etc., The use of the compounds according to the invention as flow improvers enables the surface tension to be lowered. The surface tension can be calculated by determining the wetting angle of a drop of liquid on a surface (contact angle measurement).
Therefore, the present invention also relates to a method for improving the flow of a curable composition on the substrate to which it is applied, which method comprises adding to the curable composition at least one polymerization initiator (I) as defined above.

A preferred embodiment of the invention relates to a process for the preparation of coatings by dual cure as described above, wherein in embodiments (1) and (3) R₃ in para position of the phenyl ring represents a substituent selected from the group consisting of R_{c}-Y-, R_{c}-Y-(CH₂)ₓ-, R_{c}-(CH₂)ₓ-Y-, R₀-Y-(CH₂)_{y}-O-, R_{c}-Y-(CH₂)_{y}-S- and R_{c}-Y-(CH₂)_{y}-NR₈-; and the other R₁, R₂, R₄ and R₅ represent hydrogen or substituents selected from the group consisting of C₁-C₄alkyl, hydroxy, hydroxy-C₂-C₄alkyl, C₁-C₄alkoxy, hydroxy-C₂-C₄alkoxy, halogen, amino and di-C₁-C₄alkylamino.

Another particularly preferred embodiment of the invention as exemplified relates to a process for the preparation of coatings by dual cure, which is **characterized in that** a composition comprising
a) at least one polymerization initiator (I),
   wherein in embodiment (1)
   - X: represents the terminal groups RₐO- or RₐR_{b}N-;
   - R₃: in para position of the phenyl ring represents a substituent selected from the group consisting of R_{c}-Y-, R_{c}-Y-(CH₂)ₓ-, R_{c}-(CH₂)ₓ-Y-, R_{c}-Y-(CH₂)_{y}-O- and R_{c}-Y-(CH₂)_{y}-S-; and
   - R₁, R₂, R₄ and R₅: represent hydrogen or substituents selected from the group consisting of C₁-C₄alkyl, hydroxy, hydroxy-C₂-C₄alkyl, C₁-C₄alkoxy and hydroxy-C₂-C₄alkoxy; or
   wherein in embodiment (2)
   - X: represents a terminal group selected from the group consisting of R_{c}-Y₁-, R_{c}-(CH₂)ₓ-Y₁- and R_{c}-Y-(CH₂)_{y}-O-;
   - R₁ - R₅: represent hydrogen or substituents selected from the group consisting of C₁-C₄alkyl, hydroxy, hydroxy-C₂-C₄alkyl, C₁-C₄alkoxy and hydroxy-C₂-C₄alkoxy; or
   wherein in embodiment (3)
   - X: represents a terminal group selected from the group consisting of R_{c}-Y₁-, R_{c}-(CH₂)ₓ-Y₁- and R_{c}-Y-(CH₂)_{y}-O-;
   - R₃: in para position of the phenyl ring represents a substituent selected from the group consisting of R_{c}-Y-, R_{c}-Y-(CH₂)ₓ-, R_{c}-(CH₂)ₓ-Y-, R_{c}-Y-(CH₂)_{y}-O- and R_{c}-Y-(CH₂)_{y}-S-;
   - R₁, R₂, R₄ and R₅: represent hydrogen or substituents selected from the group consisting of C₁-C₄alkyl, hydroxy, hydroxy-C₂-C₄alkyl, C₁-C₄alkoxy and hydroxy-C₂-C₄alkoxy; and
   wherein in embodiment (1), (2) and (3)
   R₆, R₇, Rₐ, R_{b}, R_{c}, x, y, Y, Y₁ are as defined above; and
b) polymerizable, ethylenically unsaturated compounds; and, optionally, as further components; a thermally crosslinkable compound; and/or further additives; and/or additional photoinitiators; is cured as described above.

A particularly preferred embodiment of the invention relates to a process for the preparation of coatings by dual cure, which is **characterized in that** a composition comprising
a) at least one polymerization initiator (I), wherein
   wherein in embodiment (1)
   - X: represents the terminal groups RₐO- or RₐR_{b}N-; and
   - R₃: in para position of the phenyl ring represents a substituent selected from the group consisting of R_{c}-(CH₂)ₓ-Y- or R_{c}-Y-(CH₂)_{y}-O- and
   the other R₁, R₂, R₄ and R₅ represent hydrogen
   wherein in embodiment (2)
   - X: represents a terminal group Rₒ-Y₁-, and
   - R₁ - R₆: represent hydrogen; or
   wherein in embodiment (3)
   - X: represents a terminal group R_{c}-Y₁-; and
   - R₃: in para position of the phenyl ring represents a substituent R_{c}-Y-(CH₂)_{y}-O-, and the other R₁, R₂, R₄ and R₅ represent hydrogen; and
   wherein in embodiment (1), (2) and (3)
   - R₆ and R₇: independently of one another are C₁-C₁₂-alkyl or benzoyl;
   - Rₐ and R_{b}: independently of one another represent hydrogen or C₁-C₁₂alkyl; or Rₐ and R_{b} together with the nitrogen atom to which they are bonded form a morpholinyl, piperidinyl or piperazinyl ring.
   - R_{c}: represents a linear or branched terminal chain ZCF₂(-O-C₂F₄)ₚ-(CF₂)_{q}-, wherein Z represents -H or -F; one of p and q represents a numeral from zero to twenty and the other one a numeral from one to twenty;
   - x: represents a numeral from one to ten;
   - y: represents a numeral from two to ten;
   - Y and Y₁: independently of one another represents a bivalent substituent selected from the group consisting of -S-, and -O-Si(R₉)₂-(CH₂)ₓ-;
   - R₉: is C₁-C₈ alkyl or phenyl.
b) polymerizable, ethylenically unsaturated compounds; and, optionally, as further components; a thermally crosslinkable compound; and/or further additives; and/or additional photoinitiators; is cured as described above.

Another highly preferred embodiment of the invention relates to a process for the preparation of coatings by dual cure and by employing the novel compounds defined below. The process is **characterized in that** a composition comprising
a) at least one polymerization initiator of the formula 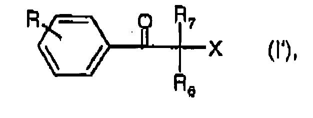 wherein in embodiment (1)
   - X: represents the terminal groups RₐO- or RₐR_{b}N-; and
   - R: represents a substituent selected from the group consisting of R_{c}-Y-, R_{c}-Y-(CH₂)ₓ-, R_{c}-(CH₂)ₓ-Y-, R_{c}-Y-(CH₂)_{y}-O-, R_{c}-Y-(CH₂)_{y}-S-; and R_{c}-Y-(CH₂)_{y}-NR₈-;or
   wherein in embodiment (2)
   - X: represents a terminal group selected from the group consisting of R_{c}-Y₁-, R_{c}-(CH₂)ₓ-Y₁- or R_{c}-Y-(CH₂)_{y}-O- ; and
   - R: represent hydrogen or a substituent selected from the group consisting of C₁-C₄alkyl, hydroxy, hydroxy-C₂-C₄alkyl, C₁-C₄alkoxy, hydroxy-C₂-C₄alkoxy; or
   wherein in embodiment (3)
   - X: represents a terminal group selected from the group consisting of R_{c}-Y₁-, R_{c}-(CH₂)ₓ-Y₁- or R_{c}-Y-(CH₂)_{y}-O-; and
   - R: represents a substituent selected from the group consisting of R_{c}-Y-, R_{c}-Y-(CH₂)ₓ-, R_{c}-(CH₂)ₓ-Y-, R_{c}-Y-(CH₂)_{y}-O-, R_{c}-Y-(CH₂)_{y}-S- and R_{c}-Y-(CH₂)_{y}-NR₈-; and
   wherein in embodiment (1), (2) (3)
   - R₆, R₇, R₈, R₉: are as defined above under formula I,
   - Rₐ, R_{b} and R_{c}: are as defined above under formula I,
   - x and y are: as defined above under formula I,
   - Y and Y₁: are as defined above under formula I,
b) polymerizable, ethylenically unsaturated compounds; and, optionally, as further components; a thermally crosslinkable compound; and/or further additives; and/or additional photoinitiators;
   is applied to a support and cured by irradiation with electromagnetic radiation of a wavelength from 200 nm up to the IR domain combined with prior, simultaneous or subsequent thermal curing.

Another embodiment of the invention relates to a process for the preparation of coatings by irradiation with electromagnetic radiation and employing the novel compounds of the formula I' defined below. The process is **characterized in that** a composition comprising
a) at least one polymerization initiator of the formula I' as defined above; and
b) polymerizable, ethylenically unsaturated compounds; and, optionally, further additives; and/or additional photoinitiators;
   is applied to a support and cured by irradiation with electromagnetic radiation of a wavelength from 200 nm up to the IR domain,

The preparation and the use of this compound In curing processes using electromagnetic radiation is described in *WO 93*/*12150*.

The present invention also relates **to novel polymerization Initiators** of the formula (I') as described above-

The compounds of formula I and I' are prepared in a manner known per se, e.g. by reacting
1) a compound of the formula 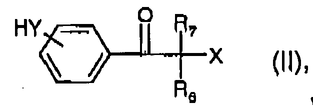 wherein
   Y represents oxygen or sulfur;
   with a reactive functional derivative introducing the substituent selected from the group consisting of R_{c}-Y-, A_{c}-Y-(CH₂)ₓ-, R_{c}-(CH₂)ₓ-Y-, R_{c}-Y-(CH₂)_{y}-O-, R_{c}-Y-(CH₂)_{y}-S-, R_{c}Y-(CH₂)_{y}-NR₈-; or
2) a compound 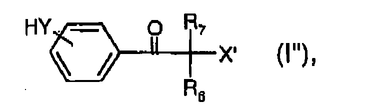 wherein
   Y represents -NH-, or sulfur;
   X' represents hydroxy or amino
   with a reactive functional derivative introducing the substituent selected from the group consisting of R_{c}-Y-, R_{c}-Y-(CH₂)ₓ-, R_{c}-(CH₂)_{y}-Y-, R_{c}-Y-(CH₂)_{y}-O-, R_{c}-Y-(CH₂)_{y}-S- and R_{c}-Y-(CH₂)_{y}-NR₈-, and, correspondingly, from the group consisting of R_{c}-Y₁-, R_{c}-(CH₂)ₓ-Y₁-and R_{c}-Y-(CH₂)_{y}-O-.

Reactive functional derivatives introducing the above-mentioned substituents are, for example the acid halides, e.g. acid chlorides or bromides, or acid anhydrides of the carboxylic acids, or alkyl or silyl halides corresponding to the above-mentioned groups. The reaction is preferably carried out in the presence of a suitable base, e.g. triethylamine or diazabicyclo[5.4.0]undec-8-ene (DBU).

Preferred embodiments of the process are illustrated in the Examples.

### (Example 7)

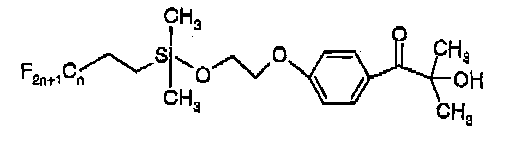

a compound of formula I' wherein
- X: RₐO,
- R: in the paraposition is R_{c}-Y-(CH₂)_{y}-O-,
- R₆ and R₇: are C₁-C₁₂alkyl, (methyl)
- Rₐ: is hydrogen,
- R_{c}: represents a linear or branched terminal chain ZCF₂(-O-C₂F₄)ₚ-(CF₂)_{q}-, wherein Z represents -F; p = zero, q= 2-20,
- y: is 2;
- Y: represents a bivalent substituent -O-Si(R₉)₂-(CH₂)ₓ-;
- R₉: is methyl,
- X: is 2.

### (Examples 8, 9 and 10)

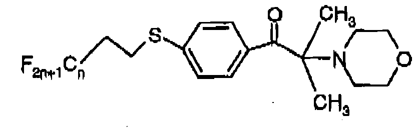

a compound of formula I' wherein
- X: RₐR_{b}N;
- R: in the paraposition is R_{c}-(CH₂)ₓ-Y-,
- R₆ and R₇: are C₁-C₁₂alkyl, (methyl)
- RₐR_{b}: together with the nitrogen atom to which they are bonded form a 6 membered ring interrupted by O, (morpholinyl)
- R_{c}: represents a linear or branched terminal chain ZCF₂(-O-C₂F₄)ₚ-(CF₂)_{q}-, wherein Z represents -F; p = zero, q= 2-20,
- x: is 2;
- Y: represents a bivalent substituent -S-.

### (Examples 11 and 12)

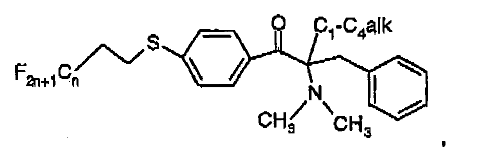

a compound of formula I' wherein
- X: RₐR_{b}N;
- R: In the paraposition is R_{c}-(CH₂)ₓ-Y-,
- R₆: is C₁-C₄alkyl,
- R₇: is benzyl,
- RₐR_{b}: are methyl,
- R_{c}: represents a linear or branched terminal chain ZCF₂(-O-C₂F₄)ₚ-(CF₂)_{q}-, wherein Z represents -F; p = zero, q= 2-20,
- x: is 2;
- Y: represents a bivalent substituent -S-.

The present invention also relates to a composition comprising
a)at least one polymerisation Initiator (I'), wherein R, R₆ and R₇ and X are as defined above,
   and
b) polymerizable, ethylenically unsaturated compounds.
c)

The component b) in these compositions is analogous to the component b) described above in regard to the compositions comprising polymerization initiators of the formula (I).

A preferred embodiment relates to compositions comprising as optional components
c) at least one thermally crosslinkable compound;
d) further additives; and
e) additional photoinitiators.

Components c), d) and e) are analogous to the components described above in regard to the compositions comprising polymerization initiators of the formula (I).

The following examples illustrate the invention without limiting the scope thereof:

### Examples

### Example 1

### Heptafluorobutyric acid 1-(4-hydroxybenzoyl)-1-methylethyl ester

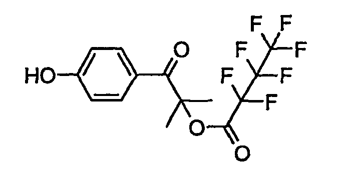

(R₁ = R₂ = R₄ = R₅ = H; R₃ = -OH; R₆ = R₇ = -CH₃; X = R_{c}-Y₁- with Y₁ = -O-C(=O)-; R_{c} = ZCF₂(-O-C₂F₄)ₚ-(CF₂)_{q}- with p = 0, q = 2, Z = F)
3.19 g (31.5 mmol, 2.03 eq.) Triethylamine and 0.1 g (0.82 mmol, 0.053 eq.) 4-dimethylaminopyridine are added at room temperature under argon atmosphere to a solution of 2.79 g (15.5 mmol, 1 eq.) 2-hydroxy-1-(4-hydroxyphenyl)-2-methyl-1-propanone in 80 ml dichloromethane. 3.95 g (17 mmol, 1.1 eq.) Heptafluorobutyric acid chloride is added within 10 min. and the solution is stirred at room temperature for 22 hours. The solvent is removed under vacuum and the residue is dissolved with 100 ml diethyl ether. The solution is treated with 100 ml 5% HCl and the aqueous phase extracted twice with diethyl ether. The combined organic phases are washed with 5% HCl (2 x 150 ml), sat. NaHCO₃ (2 x 200 ml) solution and brine (2 x 200 ml). The organic phase is dried over MgSO₄ and evaporated under vacuum. A white solid is obtained which is recrystallized from hexane giving 4.71 g (81%) of the title compound (CAS nomenclature: 3,3,4,4,4-heptafluoro-butyric acid 2-(4-hydroxy-phenyl)-1,1-dimethyl-2-oxo-ethyl ester) as a white solid.
M.p.: 89°C; UV (CH₃CN): max. at 279 nm (e: 12 797); ¹H-NMR (CDCl₃): 7.85 (m, 2 H arom.), 6.79 (m, 2 H arom.), 1.75 (s, 6 H, 2 CH₃); M/z (EI): 376 (M⁺);

| Elemental analysis for C₁₄H₁₁F₇O₄: | | | |
|---|---|---|---|
| | %C | %H | %F |
| calc. | 44.69 | 2.95 | 35.35 |
| found | 45.90 | 2.85 | 34.68 |

### Example 2

### Heptafluorobutyric acid 1-(4-methoxybenzoyl)-1-methylethyl ester

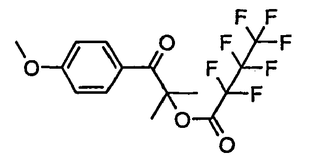

(R₁ = R₂ = R₄ = R₅ = H; R₃ = -OCH₃; R₆ = R₇ = -CH₃; X = R_{c}-Y₁- with Y₁ = -O-C(=O)-; R_{c} = ZCF₂(-O-C₂F₄)ₚ-(CF₂)_{q}- with p = 0, q = 2, Z = F)
2.5 g (6.65 mmol) of the product from Example 1 are dissolved in a mixture of THF/1,3-dimethyl-3,4,5,6-tetrahydro-2(1H)-pyrimidone (DMPU, 80 ml, 1:4) and added under argon atmosphere within 1 hour to a suspension of 0.32 g (7.31 mmol, 1.1 eq.) NaH (55-65% dispersion in oil, in a mixture of THF/DMPU (19 ml, 1 : 4). A solution of 1.04 g (7.31 mmol, 1.1 eq.) methyl iodide in 10 ml THF/DMPU (1 : 4) is added within 30 minutes. The mixture is stirred for 24 hours at room temperature, poured on 100 ml water and extracted with tert.-butyl methyl ether (TBME). After washing with water the organic phase is dried over MgSO₄ and evaporated under vacuum The yellow liquid obtained is purified by flash chromatography (FC). 0.65 g (25%) of the title compound (CAS nomenclature: 2,2,3,3,4,4,4-heptafluoro-butyric acid 2-(4-methoxy-phenyl)-1,1-dimethyl-2-oxo-ethyl ester) is obtained as a colorless liquid.
UV (CH₃CN): max. at 280 nm (e: 16 607); ¹H-NMR (CDCl₃): 7.84 (m, 2 H arom.), 6.78 (m, 2 H, arom.), 3.73 (s, 3 H, O-CH₃), 1.75 (s, 6 H, 2 CH₃).

### Example 3

### Pentadecafluorooctanoic acid 1-(4-hydroxybenzoyl)-1-methylethyl ester

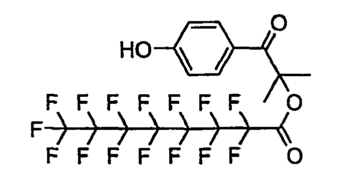

(R₁ = R₂ = R₄ = R₅ = H; R₃ = -OH; R₆ = R₇ = -CH₃; X = R_{c}-Y₁- with Y₁ = -O-C(=O)-; R_{c} = ZCF₂(-O-C₂F₄)ₚ-(CF₂)_{q}- with p = 0, q = 6, Z = F)

The title compound is prepared in a manner analogous to Example 1 by using a 1 molar equivalent of 2-hydroxy-1-(4-hydroxyphenyl)-2-methyl-1-propanone and 1.1 molar equivalent of pentadecafluorooctanoyl chloride.
UV (CH₃CN): max. at 277 nm (e: 14 713); ¹H-NMR (CDCl₃): 7.95 (m, 2 H arom.), 6.82 (m, 2 H arom.), 1.56 (s, 6 H, 2 CH₃); M/z (CI): 577 (MH⁺);

| Elemental analysis for C₁₈H₁₁F₁₅O₄: | | | |
|---|---|---|---|
| | %C | %H | %F |
| calc. | 37.52 | 1.92 | 49.45 |
| found | 37.35 | 1.69 | 49.33 |

### Example 4

### Pentadecafluorooctanoic acid 1-(4-methoxybenzoyl)-1-methylethyl ester

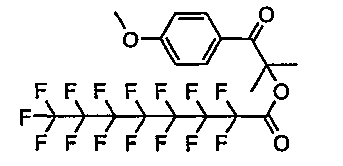

(R₁ = R₂ = R₄ = R₅ = H; R₃ = -OCH₃; R₆ = R₇ = -CH₃; X = R_{c}-Y₁- with Y₁ = -O-C(=O)-; R_{c} = ZCF₂(-O-C₂F₄)ₚ-(CF₂)_{q}- with p = 0, q = 6, Z = F)
The title compound is prepared in a manner analogous to Example 3 by methylation of the product from Example 3.
UV (CH₃CN): max. at 279 nm (e: 15 908); ¹H-NMR (CDCl₃): 8.06 (m, 2 H arom.), 6.94 (m, 2 H arom.), 3.88 (s, 3 H, O-CH₃), 1.64 (s, 6 H, 2 CH₃).

### Example 5

### Heptafluorobutyric acid 2-[4-(2-hdroxy-2-methyl-propionyl)-phenoxy]-ethyl ester (5a) and heptafluorobutyric acid 1-[4-(2-heptafluorobutyryloxy)-ethoxybenzoyl]-1-methylethyl ester (5b)

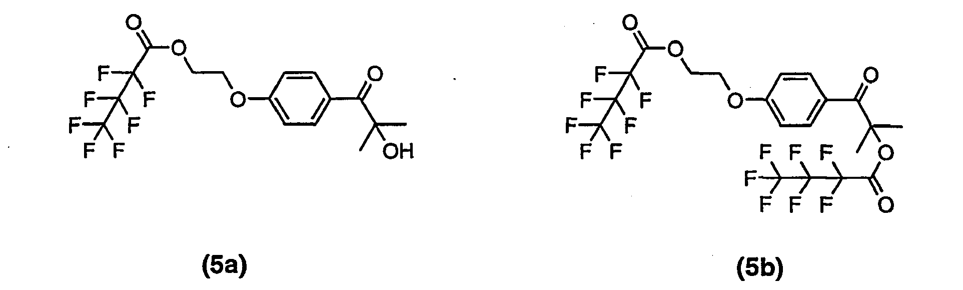

(**5a :** R₁ = R₂ = R₄ = R₅ = H; R₃ = -O-(CH₂)_{y}-Y-R_{c} ; y = 2; R₆ = R₇ = -CH₃; X = -OH; Y= -O-C(=O)-; R_{c} = ZCF₂(-O-C₂F₄)ₚ-(CF₂)_{q}- with p = 0, q = 2, Z = F)
(**5b** : R₁ = R₂ = R₄ = R₅ = H; R₃ = -O-(CH₂)_{y}-Y-R_{c}; y = 2; R₆ = R₇ = -CH₃; X = -Y₁-R_{c}; Y= Y₁ = -O-C(=O)-; R_{c} = ZCF₂(-O-C₂F₄)ₚ-(CF₂)_{q}- with p = 0, q = 2, Z = F)
The mixture of title compounds is prepared in a manner analogous to Example 1 by using 1 molar equivalent of 2-hydroxy-1-[4-(2-hydroxyethoxy)-phenyl]-2-methyl-propan-1-one and 1.1 molar equivalents of heptafluorobutyric acid chloride.
**5a** (CAS nomenclature: 2,2,3,3,4,4,4-heptafluoro-butyric acid 2-[4-(2-hydroxy-2-methylpropionyl)-phenoxy]-ethyl ester)
UV (CH₃CN): max. at 270 nm (e: 15 470); ¹H-NMR (CDCl₃): 8.06 (m, 2 H arom.), 6.92 (m, 2 H arom.), 4.74 (m, 2 H, CH₂-O-Ph), 4.32 (m, 2 H, CH₂-CH₂-O-Ph), 1.60 (s, 6 H, 2 CH₃); M/z (CI): 421 (MH⁺).
**5b** (CAS nomenclature: 2,2,3,3,4,4,4-heptafluoro-butyric acid 2-{4-[2-(2,2,3,3,4,4,4-heptafluoro-butyryloxy)-ethoxy]-phenyl}-1,1-dimethyl-2-oxo-ethyl ester)
UV (CH₃CN): max. at 276 nm (e: 16 610); ¹H-NMR (CDCl₃): 8.16 (m, 2 H arom.), 7.10 (m, 2 H arom.), 4.94 (m, 2 H, CH₂-O-Ph), 4.50 (m, 2 H, CH₂-CH₂-O-Ph), 2.02 (s, 6 H, 2 CH₃); M/z (CI): 617 (MH⁺).

### Example 6

### Pentadecafluorooctanoic acid 2-[4-(2-hydroxy-2-methyl-propionyl)-phenoxy]-ethyl ester (6a), pentadecafluorooctanoic acid 1-(4-(2-hydroxyethoxybenzoyl)-1-methylethyl ester (6b) and pentadecafluorooctanoic acid 1-[4-(2-pentadecafluorooctanoyloxy)-ethoxybenzoyl]-1-methylethyl ester (6c)

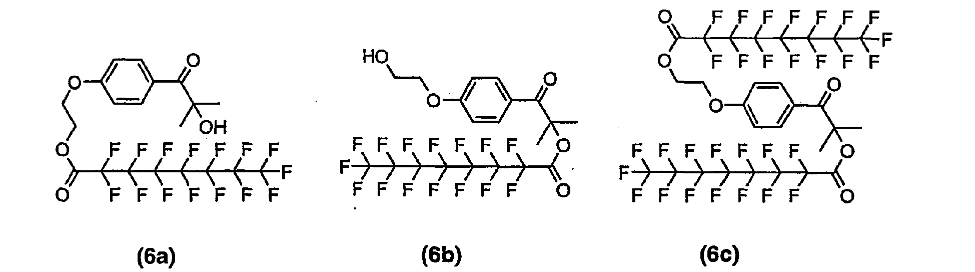

(**6a** : R₁ = R₂ = R₄ = R₅ = H; R₃ = -O-(CH₂)_{y}-Y-R_{c}; y = 2; R₆ = R₇ = -CH₃; X = -OH; Y= -O-C(=O)-; R_{c} = ZCF₂(-O-C₂F₄)ₚ-(CF₂)_{q}- with p = 0, q = 6, Z = F)
(**6b** : R₁ = R₂ = R₄ = R₅ = H; R₃ = -O-(CH₂)_{y}-OH; y = 2; R₆ = R₇ = -CH₃; X = Y₁-R_{c}; Y₁ = -O-C(=O)-; R_{c} = ZCF₂(-O-C₂F₄)ₚ-(CF₂)_{q}- with p = 0, q = 6, Z = F)
(**6c :** R₁ = R₂ = R₄ = R₅ = H; R₃ = -O-(CH₂)_{y}-Y-R_{c}; y = 2; R₆ = R₇ = -CH₃; X = -Y₁-R_{c}; Y= Y₁ = -O-C(=O)-; R_{c} = ZCF₂(-O-C₂F₄)ₚ-(CF₂)_{q}- with p = 0, q = 6, Z = F)
The mixture of title compounds is prepared in a manner analogous to Example 1 by using 1 molar equivalent of 2-hydroxy-1-[4-(2-hydroxyethoxy)-phenyl]-2-methyl-propan-1-one and 1.1 molar equivalents of pentadecafluorooctanoyl chloride.
**6a** (CAS nomenclature: 2,2,3,3,4,4,5,5,6,6,7,7,8,8,8-pentadecafluoro-octanoic acid 2-[4-(2-hydroxy-2-methyl-propionyl)-phenoxy]-ethyl ester)
UV (CH₃CN): max. at 269 nm (e: 15 837); ¹H-NMR (CDCl₃): 8.00 (m, 2 H arom.), 6.87 (m, 2 H arom.), 4.69 (m, 2 H, CH₂-O-Ph), 4.26 (m, 2 H, CH₂-CH₂-O-Ph), 1.56 (s, 6 H, 2 CH₃); M/z (CI): 621 (MH⁺).
**6b** (CAS nomenclature: 2,2,3,3,4,4,5,5,6,6,7,7,8,8,8-pentadecafluoro-octanoic acid 2-[4-(2-hydroxy-ethoxy)-phenyl]-1,1-dimethyl-2-oxo-ethyl ester)
UV (CH₃CN): max. at 279 nm (e: 19 357); ¹H-NMR (CDCl₃): 7.88 (m, 2 H arom.), 6.85 (m, 2 H arom.), 4.06 (m, 2 H, CH₂-O-Ph), 3.93 (m, 2 H, CH₂-CH₂-O-Ph), 1.73 (s, 6 H, 2 CH₃); M/z (CI): 621 (MH⁺).

### Example 7

### 1-{4-[2-((3,3,4,4,5,5,6,6,7,7,8,8,9,9,10,10,10-Heptadecafluorodecyl)-dimethylsilyloxy)-ethoxy]-phenyl}-2-hydroxy-2-methyl-propan-1-one

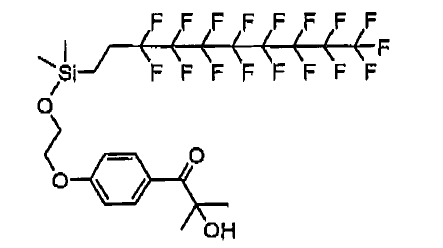

(R₁ = R₂ = R₄ = R₅ = H; R₃ = -O-(CH₂)_{y}-Y-R_{c}; y = 2; R₆ = R₇ = -CH₃; X = -OH; Y= -O-Si(R₉)₂-(CH₂)ₓ-; x = 2; R₉ = -CH₃; R_{c} = ZCF₂(-O-C₂F₄)ₚ-(CF₂)_{q}- with p = 0, q = 7, Z = F)
The title compound is prepared in a manner analogous to Example 1 by using 1 molar equivalent of 2-hydroxy-1-[4-(2-hydroxy-ethoxy)-phenyl]-2-methyl-propan-1-one and 1 molar equivalent of chloro-(3,3,4,4,5,5,6,6,7,7,8,8,9,9,10,10,10-heptadecafluorodecyl)-dimethylsilane.
UV (CH₃CN): max. at 274 nm (e: 15 500); ¹H-NMR (CDCl₃): 7.86 (m, 2 H arom.), 6.74 (m, 2 H arom.), 4.56 (s, OH), 3.92 (m, 2 H, CH₂-O-Ph), 3.80 (m, 2 H, CH₂-CH₂-O-Ph), 1.90 (m, 2 H, CH₂-CH₂-Si), 1.42 (s, 6 H, 2 CH₃), 0.64 (m, 2 H, CH₂-Si), 0.01 (s, 6 H, 2 CH₃-Si); M/z (CI): 729 (MH⁺).

### Example 8

### 1-[4-(3,3,4,4,5,5,6,6,7,7,8,8.9,9,10,10,10-Heptadecafluorodecylthio)-phenyl]-2-methyl-2-morpholin-4-yl-propan-1-one

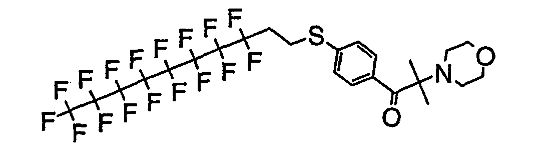

(R₁ = R₂ = R₄ = R₅ = H; R₃ = -Y-(CH₂)ₓ-R_{c}; x = 2; R₆ = R₇ = -CH₃; X = -NRₐR_{b}; -Rₐ-R_{b}- = (CH₂)₂-O-(CH₂)₂-; Y= -S-; R_{c} = ZCF₂(-O-C₂F₄)ₚ-(CF₂)_{q}- with p = 0, q = 7, Z = F)
10.7 g (40 mmol) 1-(4-Chlorophenyl)-2-methyl-2-morpholin-4-yl-propan-1-one and 23.0 g (48 mmol) Lodyne® 921 BI (C₈F₁₇-CH₂-CH₂-SH ) are dissolved in 50 ml dimethylacetamide in a 200 ml flask. 11 g K₂CO₃ are added and the suspension is heated for 18 h to 90°C. The suspension is then mixed with water and toluene. After separating off the water, the organic phase is diluted with ether and extracted several times with 2N HCl-solution. After evaporating the solvent the black residue is diluted in warm ethanol and treated with active charcoal. The crystals obtained are recrystallized from ethanol and melt at 83-86°C.

| Elemental analysis for C₂₄H₂₂F₁₇NO₂S: | | | | | |
|---|---|---|---|---|---|
| | % C | % H | %N | % S | %F |
| calc. | 40.52 | 3.12 | 1.97 | 4.50 | 45.39 |
| found | 38.25 | 2.70 | 1.80 | 5.73 | 49.30 |

### Example 9

### 2-Methyl-2-morpholin-4-yl-1-[4-(3,3,4,4,5,5,6,6,7,7,8,8,8-tridecafluorooctylthio)-phenyl]-propan-1-one

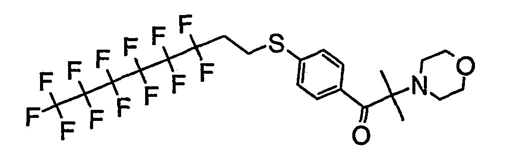

(R₁ = R₂ = R₄ = R₅ = H; R₃ = -Y-(CH₂)ₓ-R_{c}; x = 2; R₆ = R₇ = -CH₃; X = -NRₐR_{b}; -Rₐ-R_{b}- = (CH₂)₂-O-(CH₂)₂-; Y= -S-; R_{c} = ZCF₂(-O-C₂F₄)ₚ-(CF₂)_{q}- with p = 0, q = 5, Z = F)
10.7 g (40 mmol) 1-(4-Chlorophenyl)-2-methyl-2-morpholin-4-yl-propan-1-one and 18.2 g (48 mmol) Lodyne® 921 A (C₆F₁₃-CH₂-CH₂-SH ) are dissolved in 50 ml dimethylacetamide in a 200 ml flask. 11 g K₂CO₃ are added and the suspension is heated for 15 h to 90°C. The suspension is then mixed with water and ether. After separating off the organic phase is extracted several times with 2N HCl-solution. The aqueous HCl containing solution is made basic with NaOH-solution and extracted with toluene. After evaporating the solvent the residue is recrystallized from isopropanol. The brownish crystals melt at 83-86°C.

| Elemental analysis for C₂₂H₂₂F₁₃NO₂S: | | | | | | |
|---|---|---|---|---|---|---|
| | %C | %H | %N | %S | %F | %CI |
| calc.: | 43.21 | 3.63 | 2.29 | 5.24 | 40.39 | - |
| found: | 43.6 | 3.6 | 2.4 | 5.2 | 39.1 | 0.5 |

### Example 10

### 1-[4-(3,3,4,4,5,5,6,6,7,7,8,8,9,9,10,10,10-Heptadecafluordecvlthio)-phenyl]-2-methyl-2-morpholin-4-yl-propan-1-one (10a) and 1-[4-(3,3,4,4,5,5,6,6,7,7,8,8,9,9,10,10,11,11,12,12,12-Heneicosafluordodecylthio)-phenyl]-2-methyl-2-morpholin-4-yl-propan-1-one (10b)

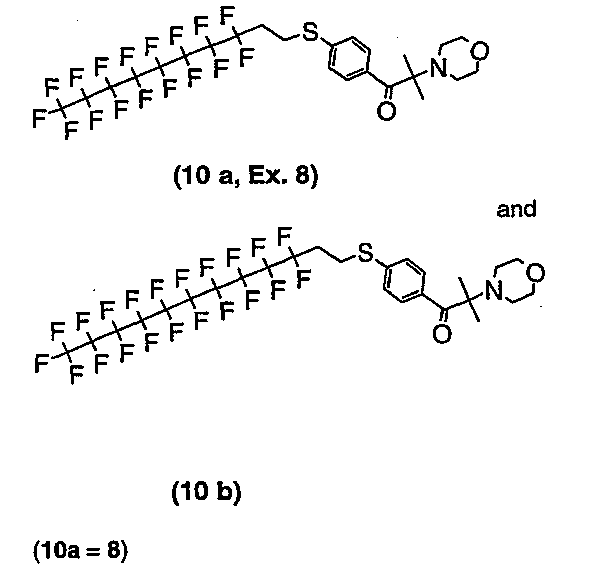

(**10b:** R₁ = R₂ = R₄ = R₅ = H; R₃ = -Y-(CH₂)ₓ-R_{c}; x = 2; R₆ = R₇ = -CH₃; X = -NRₐR_{b}; -Rₐ-R_{b}- = -(CH₂)₂-O-(CH₂)₂-; Y= -S-; R_{c} = ZCF₂(-O-C₂F₄)ₚ-(CF₂)_{q}- with p = 0, q = 9, Z = F)
10.7 g (40 mmol) 1-(4-fluororophenyl)-2-methyl-2-morpholin-4-yl-propan-1-one and 23.0 g (48 mmol) Lodyne® 926 (mixture of C₈F₁₇-CH₂-CH₂-SH and C₁₀F₂₁-CH₂-CH₂-SH) are dissolved in 150 ml dimethylsulfoxide in a 200 ml flask. 5.5 g K₂CO₃ are added and the suspension is heated for 77 h to 110°C. The suspension is then mixed with water and toluene. After separating off the organic phase is extracted several times with 2N HCl-solution. The aqueous HCl containing solution is made basic with NaOH-solution and extracted with toluene. After evaporating the solvent yellowish wax-type crystals are obtained.

| Elemental analysis for C₂₅H₂₂F₁₉NO₂S (mean value of **10a** and **10b):** | | | | | |
|---|---|---|---|---|---|
| | %C | %H | %N | %S | %F |
| calc.: | 39.43 | 2.91 | 1.84 | 4.21 | 47.40 |
| found: | 39.60 | 2.95 | 2.10 | 4.22 | 47.00 |

### Example 11

### 2-Benzyl-2-dimethylamino-1-[4-(3,3,4,4,5,5,6,6,7,7,8,8,9,9,10,10,10-heptadecafluorodecylthio)-phenyl]-butan-1-one

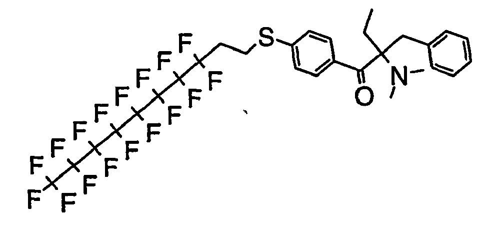

(R₁ = R₂ = R₄ = R₅ = H; R₃ = -Y-(CH₂)ₓ-R_{c}; x = 2; R₆ = -CH₂₇CH₃; = R₇ = -CH₂-C₆H₅; X = -NRₐR_{b}; Rₐ = R_{b} = -CH₃; Y= -S-; R_{c} = ZCF₂(-O-C₂F₄)ₚ-(CF₂)_{q}- with p = 0, q = 7, Z = F)
5.9 g (8,8 mmol ) 2-Dimethylamino-1-[4-(3,3,4,4,5,5,6,6,7,7,8,8,9,9,10,10,10-heptadecafluordecylthio)-phenyl]-butan-1-one are dissolved in a 100 ml flask in 20 ml methylethylketone and heated to 50°C. 1.58 g Benzyl bromide are added and the solution is stirred for 5 h at 50°C. 0.7 g sodium hydroxide are added as solid particles and the mixture is stirred for further one and a half hours. After work-up with water the orange oil is dissolved in a mixture of hexane and acetic acid ester (5 : 1), filtrated in fractions over a silica gel layer and dried. 4.0 g are obtained as a yellowish oil.

| Elemental analysis for C₂₉H₂₆F₁₇NO₂S: | | | | | |
|---|---|---|---|---|---|
| | %C | %H | %N | %S | %F |
| calc.: | 45.86 | 3.45 | 1.84 | 4.22 | 42.52 |
| found: | 47.15 | 3.72 | 1.81 | 4.28 | |

*The starting materials are prepared as follows:*
a) 2-Dimethylamino-1-[4-(3,3,4,4,5,5,6,6,7,7,8,8,9,9,10,10,10-heptadecafluorodecylthio)-phenyl]-butan-1-one
   15.0 g (20 mmol) 2-Bromo-1-[4-(3,3,4,4,5,5,6,6,7,7,8,8,9,9,10,10,10-heptadecafluordecylthio)-phenyl]-butan-1-one and 4,1 g K₂CO₃ are heated to 50°C in a 350 ml flask. 1.2 g Dimethylamine gas are fed into the mixture for 10 minutes and the suspension is stirred for 2 h at 50°C. 1.2 g Dimethylamine gas are again introduced into the mixture for 10 minutes. The reaction mixture is worked up with water after stirring for one and a half hours at 50°C. The oily liquid is oil is dissolved in a mixture of hexane and acetic acid ester (3 : 1) and purified with silica gel over a Flash column. 5.9 g of a yellowish oily liquid are obtained.
b) 2-Bromo-1-[4-(3,3,4,4,5,5,6,6,7,7,8,8,9,9,10,10,10-heptadecafluorodecylthio)-phenyl]-2-butan-1-one
   14.3 g (22.8 mmol) 1-[4-(3,3,4,4,5,5,6,6,7,7,8,8,9,9,10,10,10-Heptadecafluordecylthio)-phenyl]-butan-1-on are dissolved in 50 ml methylene chloride. 10 drops or chlorosulfonic acid are added and 3.64 g (22.8 mmol) bromine are dropped to the mixture within 15 minutes. After stirring the reaction mixture for 9 hours at room temperature the solvent is removed. 15.9 g of a black residue are obtained.
c) 1-[4-(3,3,4,4,5,5,6,6,7,7,8,8,9,9,10,10,10-Heptadecafluorodecylthio)-phenyl]-butan-1-one
   9.13 g (50 mmol) 1-(4-chlorphenyl)-butan-1-one and 24.0 g (60 mmol) Lodyne® 921BI (C₈F₁₇-CH₂-CH₂-SH) are dissolved in 100 ml dimethylacetamide in a 350 ml flask. After addition of 13.8 g K₂CO₃ the suspension is heated for 29 h to 90°C. After adding water and toluene the organic phase is removed from the suspension. After removal of the solvent the black crystals obtained are dissolved in ethyl acetate and purified over active charcoal. After removal of the solvent the brownish crystals are recrystallized with hexane. 14.6 g of crystals are obtained.

### Example 12

### 2-Benzyl-2-dimethylamino-1-[4-(3,3,4,4,5,5,6,6,7,7,8,8,8-tridecafluorooctylthio)-phenyl]-butan-1-one

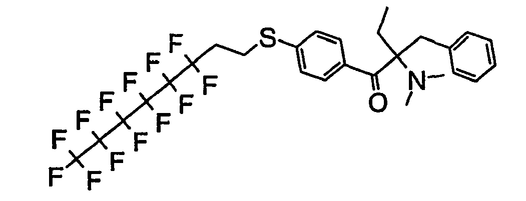

(R₁ = R₂ = R₄ = R₅ = H; R₃ = -Y-(CH₂)ₓ-R_{c}; x = 2; R₆ = -CH₂-CH₃; R₇ = -CH₂-C₆H₅; X = -NRₐR_{b}; Rₐ = R_{b} = -CH₃; Y= -S-; R_{c} = ZCF₂(-O-C₂F₄)ₚ-(CF₂)_{q}- with p = 0, q = 5, Z = F)
8.9 g (15.6 mmol) 2-Dimethylamino-1-[4-(3,3,4,4,5,5,6,6,7,7,8,8,8-tridecafluoroctylthio)-phenyl]-2-dimethylamino-butan-1-one are dissolved in a 100 ml flask in 20 ml methylethylketone and heated to 50°C. 3.78 g Benzyl bromide are added and the solution is stirred for 5 h at 50°C. 0.7 g sodium hydroxide are added as solid particles and the mixture is stirred for further one and a half hours. After work-up with water and methylethylketone the brownish oil is dissolved in a mixture of hexane and acetic acid ester (20 : 1), filtrated in fractions over a silica gel layer and dried. 5.8 g are obtained as a waxy substance.

| Elemental analysis for C₂₇H₂₆F₁₃NO₂S: | | | | | |
|---|---|---|---|---|---|
| | %C | %H | %N | %S | %F |
| calc.: | 49.17 | 3.97 | 2.12 | 4.86 | 37.45 |
| found: | 49.32 | 4.06 | 1.97 | 4.96 | |

### The starting materials are prepared as follows:

a) 2-Dimethylamino-1-[4-(3,3,4,4,5,5,6,6,7,7,8,8,8-tridecafluoroctylthio)-phenyl]-butan-1-one
   18.76 g (31 mmol) 2-Bromo-1-[4-(3,3,4,4,5,5,6,6,7,7,8,8,8-tridecafluoroctylthio)-phenyl]-butan-1-one and 6.4 g K₂CO₃ are heated to 50°C in a 350 ml flask. 1.82 g Dimethylamine gas are fed into the mixture for 10 minutes and the suspension is stirred for 2 h at 50°C. 1.0 g Dimethylamine gas are again introduced into the mixture for 10 minutes. The reaction mixture is worked up with water and methylethylketone after stirring for 1 h at 50°C. The oily liquid is dissolved in a mixture of hexane and acetic acid ester (3 : 1) and purified with silica gel over a Flash column. 8.9 g of a black oily liquid are obtained.
b) 2-Bromo-1-[4-(3,3,4,4,5,5,6,6,7,7,8,8,8-tridecafluoroctylthio)-phenyl]-butan-1-one
   16.7 g (31 mmol) 1-[4-(3,3,4,4,5,5,6,6,7,7,8,8,8-Tridecafluoroctylthio)-phenyl]-butan-1-one are dissolved in 50 ml methylene chloride. 10 drops or chlorosulfonic acid are added and 4.95 g (31 mmol) bromine are dropped to the mixture within 15 minutes. After stirring the reaction mixture for 3 hours at room temperature the solvent is removed. 19.0 g of a black residue are obtained.

### Example 13

### Curing of a UV/thermally curable system (Dual Cure)

A "dual cure" clear lacquer is prepared by mixing the following components:

| | |
|---|---|
| 21.1 parts | hydroxy functional polyacrylate (Desmophen® LS 2009/1, Bayer AG); |
| 32.3 parts | isocyanurate based urethane acrylate, 80% in butyl acetate (Roskydal® FWO 2518C, Bayer AG); |
| 0.3 parts | flow improver, 10% in Xylene (Baysilone® OL 17, Bayer AG); |
| 0.3 parts | flow improver (Modaflow®, Monsanto); |
| 26.0 parts | 1-methoxy-2-propanol, (Fluka Chemicals); |
| 0.5 parts | flow improver (Byk® 306, Byk-Chemie); |
| 11.2 parts | urethane acrylate with isocyanate groups (Roskydal® FWO 2545 E, Bayer AG). |

The samples are prepared by adding 2% of the photoinitiator 1-{4-[2-((3,3,4,4,5,5,6,6,7,7,8,8,9,9,10,10,10-heptadecafluorodecyl)-dimethylsilyloxy)-ethoxy]-phenyl}-2-hydroxy-2-methyl-propan-1-one (Example 7).
The mixture is applied to a white coil-coat aluminum, air-dried for 5 minutes at room temperature and heated for 10 minutes on a hot plate at 80 °C. The irradiation is carried out by using a UV-processor (Hg medium pressure lamps, 2x120 W/cm) at a belt speed of 5 m/min. A tack free dry film with a thickness of approximately 40 µ is obtained. 45 Minutes after cure, the pendulum hardness according to König (DIN 53157) is determined. The Surface energy of the coating is determined by measuring static water contact angle (θ) using a contact angle measuring system G10 from Krüss (Krüss User Manual, Drop Shape Analysis, Krüss GmbH, DE-Hamburg 1997) The higher the values of the pendulum hardness measurement, the harder is the cured surface. The higher the contact angle, the better is the moisture resistance and scratch resistance.

| Initiator | Pendulum Hardness [sec] | Water Contact Angle θ |
|---|---|---|
| 2% DAROCUR 1173 | 85 | 81 |
| Photoinitiator from Ex. 7 | 88 | 87 |

### Example 14

### Curing of a UV/thermally curable system (Dual Cure)

A "dual cure" clear lacquer is prepared by mixing the components from Example 13. The samples are prepared by adding 2% of the photoinitiator 2-benzyl-2-dimethylamino-1-[4-(3,3,4,4,5,5,6,6,7,7,8,8,9,9,10,10,10-heptadecafluorodecylthio)-phenyl]-butan-1-one from Example 11. The "dual cure" step of the mixture and the determination of the pendulum hardness and contact angle is carried out in a manner analogous to Example 13.

| Initiator | Pendulum hardness [sec] | water contact angle θ |
|---|---|---|
| 2% IRGACURE 369 | 90 | 80 |
| 2% from Example 11 | 76 | 89 |

## Claims

1. A process for the preparation of coatings which is **characterized in that** a composition comprising
a) at least one polymerization initiator of the formula 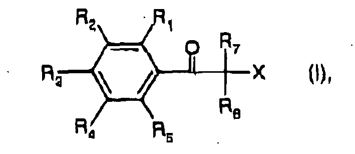 wherein in embodiment (1)
X represents the terminal groups RₐO- or RₐR_{b}N-; and one or two of
R₁ - R₅ represent substituents selected from the group consisting of R_{c}-Y-, R_{c}-Y-(CH₂)ₓ-, R_{c}-(CH₂)ₓ-Y-, R_{c}-Y-(CH₂)_{y}-O-, R_{c}-Y-(CH₂)_{y}-S- and
R_{c}-Y-(CH₂)_{y}-NR₈-; and the other R₁ - R₅ represent hydrogen or substituents selected from the group consisting of C₁-C₄alkyl, hydroxy, hydroxy-C₂-C₄alkyl, C₁-C₄alkoxy, hydroxy-C₂-C₄alkoxy, halogen, amino and di-C₁-C₄alkylamino; or
wherein in embodiment (2)
X represents a terminal group selected from the group consisting of R_{c}-Y₁-, R_{c}-(CH₂)ₓ-Y₁-, R_{c}-Y-(CH₂)_{y}-O- and R_{c}-Y-(CH₂)_{y}-NR₈-; and
R₁ - R₆ represent hydrogen or substituents selected from the group consisting of C₁-C₄alkyl, hydroxy, hydroxy-C₂-C₄alkyl, C₁-C₄alkoxy, hydroxy-C₂-C₄alkoxy, halogen, amino and di-C₁-C₄alkylamino; or
wherein in embodiment (3)
X represents a terminal group selected from the group consisting of R_{c}-Y₁-, R_{c}-(CH₂)ₓ-Y₁-, R_{c}-Y-(CH₂)_{y}-O- and R_{c}-Y-(CH₂)_{y}-NR₈-; and one or two of
R₁ - R₆ represent substituents selected from the group consisting of R_{c}-Y-, R_{c}-Y-(CH₂)ₓ-, R_{c}-(CH₂)ₓ-Y-, R_{c}-Y-(CH₂)_{y}-O-, R_{c}-Y-(CH₂)_{y}-S- and R_{c}-Y-(CH₂)_{y}-NR₈-;and the other R₁ - R₆ represent hydrogen or substituents selected from the group consisting of C₁-C₄alkyl, hydroxy, hydroxy-C₂-C₄alkyl, C₁-C₄alkoxy, hydroxy-C₂C₄alkoxy, halogen, amino and di-C₁-C₄alkylamino; and
wherein in embodiment (1), (2) and (3)
R₆ and R₇ independently of one another represent C₁-C₁₂alkyl, C₂-C₈alkenyl, C₅-C₈cycloalkyl or phenyl-C₁-C₃alkyl; or R₈ and R₇ together represent C₂-C₈alkylen, C₃-C₉oxaalkylene or C₃-C₈-azaalkylene;
Rₐ and R_{b} independently of one another represent hydrogen, C₁-C₁₂alkyl or C₂-C₆alkenyl; or Rₐ and R_{b} together represent C₄-C₆alkylene and together with the nitrogen atom to which they are bonded form a 5- or 6-membered ring, which may be interrupted by -O- or by -N-R₈-;
R_{c} represents a linear or branched terminal chain ZCF₂(-O-C₂F₄)ₚ-(CF₂)_{q}-, wherein Z represents -H or -F; one of p and q represents a numeral from zero to twenty and the other one a numeral from one to twenty;
x represents a numeral from one to ten;
y represents a numeral from two to ten;
Y represents a bivalent substituent selected from the group consisting of -S-, -NR₈-,-Si(R₉)₂-, -Si(R₉)₂-O-, -O-Si(R₈)₂-O- and -O-Si(R₉)₂-(CH₂)ₓ-;
Y₁ represents a bivalent substituent selected from the group consisting of -NR₈-, -O-Si(R₉)₂-, -O-Si(R₉)₂-O- and -O-Si(R)₂-(CH₂)ₓ-;
R₈ represents -H or C₁-C₁₂alkyl; and
R₉ represents C₁-C₁₂alkyl or phenyl;
and
b) polymerizable, ethylenically unsaturated compounds; and, optionally, as further components; a thermally crosslinkable compound: and/or further additives; and/or additional photoinitiators;
is applied to a support and cured by irradiation with electromagnetic radiation of a wavelength from 200 nm up to the IR domain combined with prior, simultaneous or subsequent thermal curing.

2. A process according to claim 1, which is **characterized in that** a composition comprising
a) at least one polymerization initiator of the formula (I),
wherein in embodiment (1)
X represents the terminal groups RₐO- or RₐR_{b}N-;
R₃ in para position of the phenyl ring represents a substituent selected from the group consisting of R_{c}-Y-, R_{c}-Y-(CH₂)ₓ-, R_{c}-(Ch₂)ₓ-Y-, R_{c}-Y-(CH₂)_{y}-O- and R_{c}-Y-(CH₂)_{y}-S-; and
R₁, R₂, R₄ and R₅ represent hydrogen or substituents selected from the group consisting of C₁-C₄alkyl, hydroxy, hydroxy-C₂-C₄alkyl, C₁-C₄alkoxy and hydroxy-C₂-C₄alkoxy; or
wherein in embodiment (2)
X represents a terminal group selected from the group consisting of R_{c}-Y₁-, R_{c}-(CH₂)ₓ-Y₁- and R_{c}-Y-(CH₂)_{y}-O-;
R₁ - R₅ represent hydrogen or substituents selected from the group consisting of C₁-C₄alkyl, hydroxy, hydroxy-C₂-C₄alkyl, C₁-C₄alkoxy and hydroxy-C₂-C₄alkoxy;
wherein in embodiment (3)
X represents a terminal group selected from the group consisting of R_{c}-Y₁-, R_{c}-(CH₂)ₓ-Y₁- and R_{c}-Y-(CH₂)_{y}-O-;
R₃ in para position of the phenyl ring represents a substituent selected from the group consisting of R_{c}-Y-, R_{c}-Y-(CH₂)ₓ-, R_{c}-(CH₂)ₓ-Y-, R_{c}-Y-(CH₂)_{y}-O- and R_{c}-Y-(CH₂)_{y}-S-;
R₁, R₂, R₄ and R₅ represent hydrogen or substituents selected from the group consisting of C₁-C₄alkyl, hydroxy, hydroxy-C₂-C₄alkyl, C₁-C₄alkoxy and hydroxy-C₂-C₄alkoxy; and
wherein in embodiment (1), (2) and (3)
R₆, R₇, Rₐ, R_{b}, R_{c}, x, y, Y. Y₁ are as defined in claim 1;
and
b) polymerizable, ethylenically unsaturated compounds; and, optionally, as further components; a thermally crosslinkable compound; and/or further additives; and/or additional photoinitiators; is applied to a support and cured as described in claim 1.

3. A process according to claim 1, which is **characterized in that** a composition comprising
a) at least one polymerization initiator of the formula (I),
wherein in embodiment (1)
X represents the terminal groups RₐO- or RₐR_{b}N-; and
R₃ in para position of the phenyl ring represents a substituent selected from the group consisting of R_{c}-(CH₂)ₓ-Y- or R_{c}-Y-(CH₂)_{y}-O- and
the other R₁, R₂, R₄ and R₅ represent hydrogen
wherein In embodiment (2)
X represents a terminal group R_{c}-Y₁-, and
R₁ - R₅ represent hydrogen; or
wherein In embodiment (3)
X represents a terminal group R_{c}-Y₁-; and
R₃ in para position of the phenyl ring represents a substituent R_{c}-Y-(CH₂)_{y}-O-, and
the other R₁, R₂, R₄ and R₅ represent hydrogen; and
wherein in embodiment (1), (2) and (3)
R₆ and R₇ independently of one another are C₁-C₁₂-alkyl or benzyl;
Rₐ and R_{b} independently of one another represent hydrogen or C₁-C₁₂alkyl; or Rₐ and R_{b} together with the nitrogen atom to which they are bonded form a morpholinyl, piperidinyl or piperazinyl ring,
R_{c} represents a linear or branched terminal chain ZCF₂(-O-C₂F₄)ₚ-(CF₂)_{q}-, wherein Z represents -H or -F; one of p and q represents a numeral from zero to twenty and the other one a numeral from one to twenty;
x represents a numeral from one to ten;
y represents a numeral from two to ten;
Y and Y₁ independently of one another represents a bivalent substituent selected from the group consisting of -S- and -O-Si(R₉)₂-(CH₂)ₓ-;
R₉ is C₁-C₈ alkyl or phenyl; and
b) polymerizable, ethylenically unsaturated compounds; and, optionally, as further components; a thermally crosslinkable compound; and/or further additives; and/or additional photoinitiators; is applied to a support and cured as described in claim 1.

4. A process according to claim 1, which is **characterized in that** a composition comprising
a) at least one polymerization initiator of the formula I' 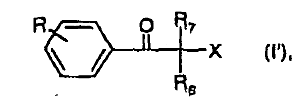 wherein in embodiment (1)
X represents the terminal groups RₐO- or RₐR_{b}N-; and
R represents a substituent selected from the group consisting of R_{c}-Y-, R_{c}-Y-(CH₂)ₓ-, R_{c}-(CH₂)ₓ-Y-, R_{c}-Y-(CH₂)_{y}-O-, R_{c}-Y-(CH₂)_{y}-S-; and R_{c}-Y-(CH₂)_{y}-NR₈-;or
wherein in embodiment (2)
X represents a terminal group selected from the group consisting of R_{c}-Y₁-, R_{c}-(CH₂)ₓ-Y₁- or R_{c}-Y-(CH₂)_{y}-O-; and
R represent hydrogen or a substituent selected from the group consisting of C₁-C₄alkyl, hydroxy, hydroxy-C₂-C₄alkyl, C₁-C₄alkoxy, hydroxy-C₂-C₄alkoxy; or
wherein in embodiment (3)
X represents a terminal group selected from the group consisting of R_{c}-Y₁-, R_{c}-(CH₂)ₓ-Y₁- or R_{c}-Y-(CH₂)_{y}-O-; and
R represents a substituent selected from the group consisting of R_{c}-Y-, R_{c}-Y-(CH₂)ₓ-, R_{c}-(CH₂)ₓ-Y-, R_{c}-Y-(CH₂)_{y}-O-, R_{c}-Y-(CH₂)_{y}-S- and R_{c}-Y-(CH₂)_{y}-NR₈-; and
wherein In embodiment (1), (2) (3)
R₆, R₇, R₈, R₉ are as defined in claim 1,
Rₐ, R_{b} and R_{c} are as defined in claim 1, x and y are as defined in claim 1,
Y and Y₁ are as defined in claim 1; and
b) polymerizable, ethylenically unsaturated compounds; and, optionally, as further components; a thermally crosslinkable compound; and/or further additives; and/or additional photoinitiators;
is applied to a support and cured as described in claim 1.

5. A process according to claim 4 which is **characterized in that** a composition comprising
a) at least one polymerization initiator of the formula I' as defined in claim 4; and
b) polymerizable, ethylenically unsaturated compounds; and, optionally, further additives; and/or additional photoinitiators;
is applied to a support and cured by irradiation with electromagnetic radiation of a wavelength from 200 nm up to the IR domain.

6. A process according to claim 1, which is **characterized in that** the composition comprises as further component
c) at least one thermally crosslinkable compound containing at least one double bond; and the curing is carried out by irradiation with electromagnetic radiation of a wavelength from 200 nm up to the IR domain combined with prior, simultaneous or subsequent thermal curing.

7. A process according to claim 6, which is **characterized in that** the thermally crosslinkable compound c) is a binder based on a composition of a polyacrylate with melamine or a melamine derivative, or a composition based on a polyacrylate polyol and a polyester polyol with an unblocked polyisocyanate or polyisocyanurate or a polyester polyol with an unblocked polyisocyanate or polyisocyanurate.

8. A process according to claim 1, for the preparation of pigmented and non-pigmented surface coatings, powder coatings, composites and glass fiber cable coatings.

9. A coated substrate that is coated on at least one surface with a composition according to claim 1.

10. A polymerization initiator of the formula (I) as described in claim 1.

11. A polymerization initiator of the formula (I) according to claim 10, selected from the group consisting of; 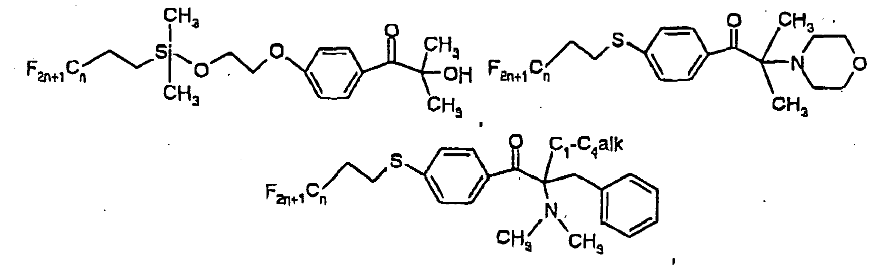 and
wherein n represents a numeral from 3 to 20.

12. A polymerization initiator of the formula (I) according to claim 10, selected from the group consisting of:

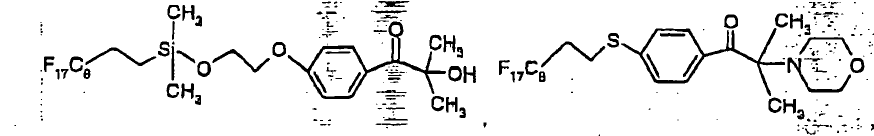

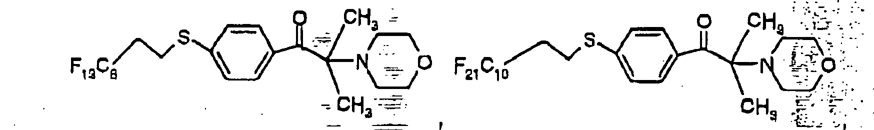

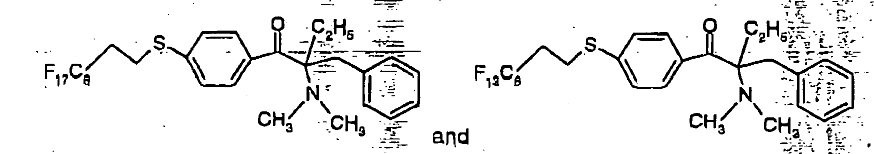

13. A composition comprising
a) at least one polymerization initiator of formula (I'), as defined in claim 4; and
b) polymerizable, ethylenically unsaturated compounds.

14. A composition according to claim 13, further comprising components selected from the group consisting of
c) at least one thermally crosslinkable compound;
d) further additives; and
e) additional photoinitiators.

## Patentansprüche

1. Verfahren zur Herstellung von Beschichtungen, das **dadurch gekennzeichnet ist, dass** eine Zusammensetzung, umfassend
a) mindestens einen Polymerisations-Starter der Formel 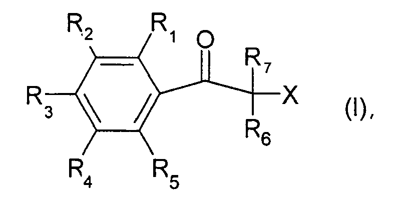 worin in Ausführungsform (1)
X die endständigen Gruppen RₐO- oder RₐR_{b}N- wiedergibt; und einer oder zwei von
R₁-R₅ Substituenten, ausgewählt aus der Gruppe, bestehend aus R_{c}-Y-, R_{c}-Y-(CH₂)ₓ-, R_{c}-(CH₂)ₓ-Y-, R_{c}-Y-(CH₂)_{y}-O-, R_{c}-Y-(CH₂)_{y}-S- und R_{c}-Y-(CH₂)_{y}-NR₈-, wiedergeben; und die anderen R₁-R₅ Wasserstoff oder Substituenten, ausgewählt aus der Gruppe, bestehend aus C₁-C₄-Alkyl, Hydroxy, Hydroxy-C₂-C₄-alkyl, C₁-C₄-Alkoxy, Hydroxy-C₂-C₄-alkoxy, Halogen, Amino und Di-C₁-C₄-alkylamino, wiedergeben; oder
worin in Ausführungsform (2)
X eine endständige Gruppe, ausgewählt aus der Gruppe, bestehend aus R_{c}-Y₁-, R_{c}-(CH₂)ₓ-Y₁-, R_{c}-Y-(CH₂)_{y}-O- und R_{c}-Y-(CH₂)_{y}-NR₈-, wiedergibt; und
R₁-R₅ Wasserstoff oder Substituenten, ausgewählt aus der Gruppe, bestehend aus C₁-C₄-Alkyl, Hydroxy, Hydroxy-C₂-C₄-alkyl, C₁-C₄-Alkoxy, Hydroxy-C₂-C₄-alkoxy, Halogen, Amino und Di-C₁-C₄-alkylamino, wiedergeben; oder
worin in Ausführungsform (3)
X eine endständige Gruppe, ausgewählt aus der Gruppe, bestehend aus R_{c}-Y₁-, R_{c}-(CH₂)ₓ-Y₁-, R_{c}-Y-(CH₂)_{y}-O- und R_{c}-Y-(CH₂)_{y}-NR₈-, wiedergibt; und einer oder zwei von
R₁-R₅ Substituenten, ausgewählt aus der Gruppe, bestehend aus R_{c}-Y-, R_{c}-Y-(CH₂)ₓ-, R_{c}-(CH₂)ₓ-Y-, R_{c}-Y-(CH₂)_{y}-O-, R_{c}-Y-(CH₂)_{y}-S- und R_{c}-Y-(CH₂)_{y}-NR₈-, wiedergeben; und die anderen R₁-R₅ Wasserstoff oder Substituenten, ausgewählt aus der Gruppe, bestehend aus C₁-C₄-Alkyl, Hydroxy, Hydroxy-C₂-C₄-alkyl, C₁-C₄-Alkoxy, Hydroxy-C₂-C₄-alkoxy, Halogen, Amino und Di-C₁-C₄-alkylamino, wiedergeben; und
worin in Ausführungsform (1), (2) und (3)
R₆ und R₇ unabhängig voneinander C₁-C₁₂-Alkyl, C₂-C₈-Al-kenyl, C₅-C₈-Cycloalkyl oder Phenyl-C₁-C₃-alkyl wieder-geben; oder R₆ und R₇ zusammen C₂-C₈-Alkylen, C₃-C₈-Oxa-alkylen oder C₃-C₉-Azaalkylen wiedergeben;
Rₐ und R_{b} unabhängig voneinander Wasserstoff, C₁-C₁₂-Alkyl oder C₂-C₆-Alkenyl wiedergeben; oder Rₐ und R_{b} zusammen C₄-C₅-Alkylen wiedergeben und zusammen mit dem Stickstoff-Atom, an das sie gebunden sind, einen 5- oder 6-gliedrigen Ring bilden, der durch -O- oder durch -N-R₈-unterbrochen sein kann;
R_{c} eine lineare oder verzweigte endständige Kette ZCF₂(-O-C₂F₄)ₚ-(CF₂)_{q}- wiedergibt, worin Z -H oder -F wiedergibt; einer von p und q eine Zahl von null bis zwanzig und der andere eine Zahl von eins bis zwanzig wiedergibt;
x eine Zahl von eins bis zehn wiedergibt;
y eine Zahl von zwei bis zehn wiedergibt;
Y einen zweiwertigen Substituenten, ausgewählt aus der Gruppe, bestehend aus -S-, -NR₈-, -Si(R₉)₂-, -Si(R₉)₂-O-, -O-Si(R₉)₂-O- und -O-Si(R₉)₂-(CH₂)ₓ-, wiedergibt;
Y₁ einen zweiwertigen Substituenten, ausgewählt aus der Gruppe, bestehend aus -NR₈-, -O-Si(R₉)₂-, -O-Si(R₉)₂-O-und -O-Si(R₉)₂-(CH₂)ₓ-, wiedergibt;
R₈ -H oder C₁-C₁₂-Alkyl wiedergibt; und
R₉ C₁-C₁₂-Alkyl oder Phenyl wiedergibt;
und
b) polymerisierbare, ethylenisch ungesättigte Verbindungen; und gegebenenfalls als weitere Komponenten eine thermisch vernetzbare Verbindung; und/oder weitere Additive; und/oder weitere Photo-Starter;
auf einen Träger aufgetragen und durch Bestrahlung mit elektromagnetischer Strahlung mit einer Wellenlänge von 200 nm bis zu der IR-Domäne, kombiniert mit vorangehender, gleichzeitiger oder anschließender Wärme-Härtung gehärtet wird.

2. Verfahren nach Anspruch 1, das **dadurch gekennzeichnet ist, dass** eine Zusammensetzung, umfassend
a) mindestens einen Polymerisations-Starter der Formel (I),
worin in Ausführungsform (1)
X die endständigen Gruppen RₐO- oder RₐR_{b}N- wiedergibt;
R₃ in para-Position von dem Phenyl-Ring einen Substituenten, ausgewählt aus der Gruppe, bestehend aus R_{c}-Y-, R_{c}-Y-(CH₂)ₓ-, R_{c}-(CH₂)ₓ-Y-, R_{c}-Y-(CH₂)_{y}-O- und R_{c}-Y-(CH₂)_{y}-S-, wiedergibt; und
R₁, R₂, R₄ und R₅ Wasserstoff oder Substituenten, ausgewählt aus der Gruppe, bestehend aus C₁-C₄-Alkyl, Hydroxy, Hydroxy-C₂-C₄-alkyl, C₁-C₄-Alkoxy und Hydroxy-C₂-C₄-alkoxy, wiedergeben; oder
worin in Ausführungsform (2)
X eine terminale Gruppe, ausgewählt aus der Gruppe, bestehend aus R_{c}-Y₁-, R_{c}-(CH₂)ₓ-Y₁- und R_{c}-Y-(CH₂)_{y}-O-, wiedergibt;
R₁-R₅ Wasserstoff oder Substituenten, ausgewählt aus der Gruppe, bestehend aus C₁-C₄-Alkyl, Hydroxy, Hydroxy-C₂-C₄-alkyl, C₁-C₄-Alkoxy und Hydroxy-C₂-C₄-alkoxy, wieder-geben; oder
worin in Ausführungsform (3)
X eine terminale Gruppe, ausgewählt aus der Gruppe, bestehend aus R_{c}-Y₁-, R_{c}-(CH₂)ₓ-Y₁- und R_{c}-Y-(CH₂)_{y}-O-, wiedergibt;
R₃ in para-Position von dem Phenyl-Ring einen Substituenten, ausgewählt aus der Gruppe, bestehend aus R_{c}-Y-, R_{c}-Y-(CH₂)ₓ-, R_{c}-(CH₂)ₓ-Y-, R_{c}-Y-(CH₂)_{y}-O- und R_{c}-Y-(CH₂)_{y}-S-, wiedergibt;
R₁, R₂, R₄ und R₅ Wasserstoff oder Substituenten, ausgewählt aus der Gruppe, bestehend aus C₁-C₄-Alkyl, Hydroxy, Hydroxy-C₂-C₄-alkyl, C₁-C₄-Alkoxy und Hydroxy-C₂C₄-alkoxy, wiedergeben; und
worin in Ausführungsform (1), (2) und (3)
R₆, R₇, Rₐ, R_{b}, R_{c}, x, y, Y, Y₁ wie in Anspruch 1 definiert sind; und
b) polymerisierbare, ethylenisch ungesättigte Verbindungen; und gegebenenfalls als weitere Komponenten eine thermisch vernetzbare Verbindung; und/oder weitere Additive; und/oder weitere Photo-Starter; auf einen Träger aufgetragen wird und wie in Anspruch 1 beschrieben, gehärtet wird.

3. Verfahren nach Anspruch 1, das **dadurch gekennzeichnet ist, dass** eine Zusammensetzung, umfassend
a) mindestens einen Polymerisations-Starter der Formel (I),
worin in Ausführungsform (1)
X die endständigen Gruppen RₐO- oder RₐR_{b}N- wiedergibt; und
R₃ in para-Position von dem Phenyl-Ring einen Substituenten, ausgewählt aus der Gruppe, bestehend aus R_{c}(CH₂)ₓ-Y- oder R_{c}-Y-(CH₂)_{y}-O-, wiedergibt und die anderen R₁, R₂, R₄ und R₅ Wasserstoff wiedergeben;
worin in Ausführungsform (2)
X eine terminale Gruppe R_{c}-Y₁- wiedergibt, und
R₁-R₅ Wasserstoff wiedergeben; oder
worin in Ausführungsform (3)
X eine terminale Gruppe R_{c}-Y₁- wiedergibt; und
R₃ in para-Position von dem Phenyl-Ring einen Substituenten R_{c}-Y-(CH₂)_{y}-O- wiedergibt, und die anderen R₁, R₂, R₄ und R₅ Wasserstoff wiedergeben; und
worin in Ausführungsform (1), (2) und (3)
R₆ und R₇ unabhängig voneinander C₁-C₁₂-Alkyl oder Benzyl darstellen;
Rₐ und R_{b} unabhängig voneinander Wasserstoff oder C₁-C₁₂-Alkyl wiedergeben; oder Rₐ und R_{b} zusammen mit dem Stickstoff-Atom, an das sie gebunden sind, einen Morpholinyl-, Piperidinyl- oder Piperazinyl-Ring bilden;
R_{c} eine lineare oder verzweigte endständige Kette ZCF₂(-O-C₂F₄)ₚ-(CF₂)_{q}- wiedergibt, worin Z -H oder -F wiedergibt; einer von p und q eine Zahl von null bis zwanzig und der andere eine Zahl von eins bis zwanzig wiedergibt;
x eine Zahl von eins bis zehn wiedergibt;
y eine Zahl von zwei bis zehn wiedergibt;
Y und Y₁ unabhängig voneinander einen zweiwertigen Substituenten, ausgewählt aus der Gruppe, bestehend aus -S- und -O-Si(R₉)₂-(CH₂)ₓ-, wiedergeben;
R₉ C₁-C₈-Alkyl oder Phenyl darstellt; und
b) polymerisierbare, ethylenisch ungesättigte Verbindungen; und gegebenenfalls als weitere Komponenten eine thermisch vernetzbare Verbindung; und/oder weitere Additive; und/oder weitere Photo-Starter; auf einen Träger aufgetragen wird und wie in Anspruch 1 beschrieben, gehärtet wird.

4. Verfahren nach Anspruch 1, das **dadurch gekennzeichnet ist, dass** eine Zusammensetzung, umfassend a) mindestens einen Polymerisations-Starter der Formel I' 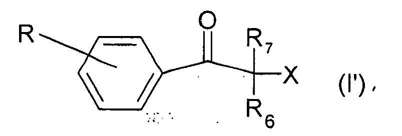 worin in Ausführungsform (1)
X die endständigen Gruppen RₐO- oder RₐR_{b}N- wiedergibt; und
R einen Substituenten, ausgewählt aus der Gruppe, bestehend aus R_{c}-Y-, R_{c}-Y-(CH₂)ₓ-, R_{c}-(CH₂)ₓ-Y-, R_{c}-Y-(CH₂)_{y}-O-, R_{c}-Y-(CH₂)_{y}-S-; und R_{c}-Y-(CH₂)_{y}-NR₈-, wiedergibt; oder
worin in Ausführungsform (2)
X eine endständige Gruppe, ausgewählt aus der Gruppe, bestehend aus R_{c}-Y₁-, R_{c}-(CH₂)ₓ-Y₁- oder R_{c}-Y-(CH₂)_{y}-O-, wiedergibt; und
R Wasserstoff oder einen Substituenten, ausgewählt aus der Gruppe, betstehend aus C₁-C₄-Alkyl, Hydroxy, Hydroxy-C₂-C₄-alkyl, C₁-C₄-Alkoxy, Hydroxy-C₂-C₄-alkoxy, wiedergeben; oder
worin in Ausführungsform (3)
X eine endständige Gruppe, ausgewählt aus der Gruppe, bestehend aus R_{c}-Y₁-, R_{c}-(CH₂)ₓ-Y₁- oder R_{c}-Y-(CH₂)_{y}-O-, wiedergibt; und
R einen Substituenten, ausgewählt aus der Gruppe, bestehend aus R_{c}-Y-, R_{c}-Y-(CH₂)ₓ-, R_{c}-(CH₂)ₓ-Y-, R_{c}-Y-(CH₂)_{y}-O-, R_{c}-Y-(CH₂)_{y}-S- und R_{c}-Y-(CH₂)_{y}-NR₈-, wiedergibt; und
worin in Ausführungsform (1), (2), (3)
R₆, R₇, R₈, R₉ wie in Anspruch 1 definiert sind,
Rₐ, R_{b} und R_{c} wie in Anspruch 1 definiert sind,
x und y wie in Anspruch 1 definiert sind,
Y und Y₁ wie in Anspruch 1 definiert sind; und
b) polymerisierbare, ethylenisch ungesättigte Verbindungen; und gegebenenfalls als weitere Komponenten eine thermisch vernetzbare Verbindung; und/oder weitere Additive; und/oder weitere Photo-Starter;
auf einen Träger aufgetragen wird und wie in Anspruch 1 beschrieben, gehärtet wird.

5. Verfahren nach Anspruch 4, das **dadurch gekennzeichnet ist, dass** eine Zusammensetzung, umfassend
a) mindestens einen Polymerisations-Starter der Formel I', wie in Anspruch 4 definiert; und
b) polymerisierbare, ethylenisch ungesättigte Verbindungen; und gegebenenfalls weitere Additive; und/oder weitere Photo-Starter;
auf einen Träger aufgetragen wird und durch Bestrahlung mit elektromagnetischer Strahlung mit einer Wellenlänge von 200 nm bis zu der IR-Domäne gehärtet wird.

6. Verfahren nach Anspruch 1, das **dadurch gekennzeichnet ist, dass** die Zusammensetzung als weitere Komponente umfasst
c) mindestens eine thermisch vernetzbare Verbindung, die mindestens eine Doppelbindung enthält;
und das Härten durch Bestrahlung mit elektromagnetischer Strahlung mit einer Wellenlänge von 200 nm bis zu der IR-Domäne, kombiniert mit vorangehender, gleichzeitiger oder anschließender Wärme-Härtung, ausgeführt wird.

7. Verfahren nach Anspruch 6, das **dadurch gekennzeichnet ist, dass** die thermisch vernetzbare Verbindung c) ein Bindemittel, basierend auf einer Zusammensetzung von einem Polyacrylat mit Melamin oder einem Melamin-Derivat, oder einer Zusammensetzung, basierend auf einem Polyacrylat-polyol und einem Polyester-polyol mit einem unblockierten Polyisocyanat oder Polyisocyanurat oder einem Polyester-polyol mit einem unblockierten Polyisocyanat oder Polyisocyanurat ist.

8. Verfahren nach Anspruch 1, für die Herstellung von pigmentierten und nicht-pigmentierten Oberflächenbeschichtungen, Pulverbeschichtungen, Verbundwerkstoffen und Glasfaserkabelbeschichtungen.

9. Beschichtetes Substrat, das auf mindestens einer Oberfläche mit einer Zusammensetzung nach Anspruch 1 beschichtet ist.

10. Polymerisations-Starter der Formel (I) wie in Anspruch 1 beschrieben.

11. Polymerisations-Starter der Formel (I) nach Anspruch 10, ausgewählt aus der Gruppe, bestehend aus: 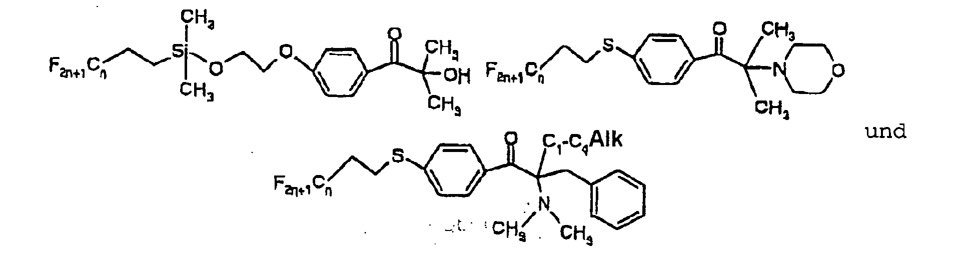 worin n eine Zahl von 3 bis 20 wiedergibt.

12. Polymerisations-Starter der Formel (I) nach Anspruch 10, ausgewählt aus der Gruppe, bestehend aus:

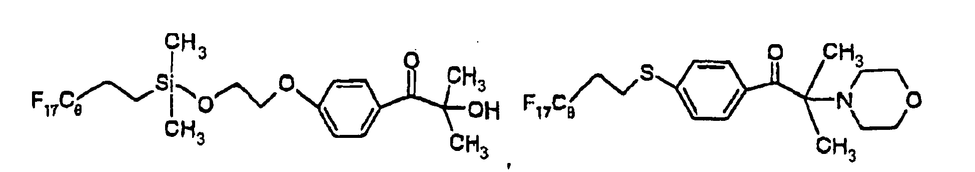

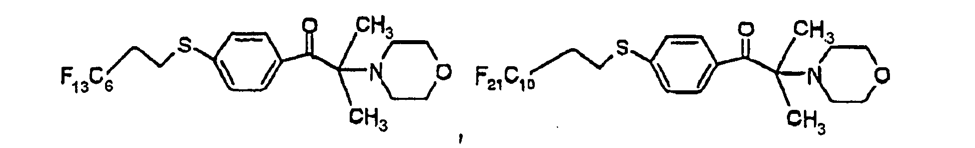

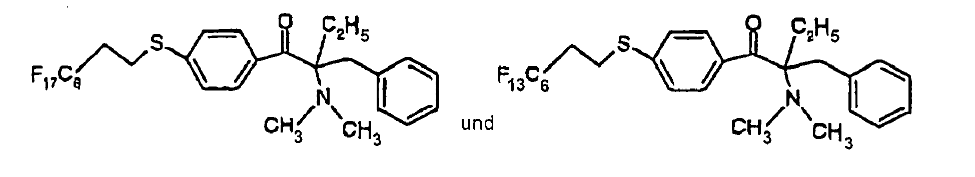

13. Zusammensetzung, umfassend
a) mindestens einen Polymerisations-Starter der Formel (I'), wie in Anspruch 4 definiert; und
b) polymerisierbare, ethylenisch ungesättigte Verbindungen.

14. Zusammensetzung nach Anspruch 13, weiterhin umfassend Komponenten, ausgewählt aus der Gruppe, bestehend aus:
c) mindestens einer thermisch vernetzbaren Verbindung;
d) weiteren Additiven; und
e) zusätzlichen Photo-Startern.

## Revendications

1. Procédé de préparation de revêtements, **caractérisé en ce qu'**une composition comprenant :
(a) au moins un initiateur de polymérisation de formule 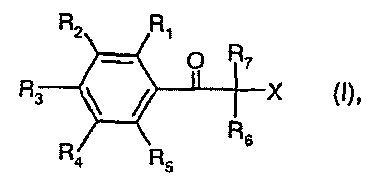 dans laquelle dans le mode de réalisation (1)
X représente un groupe terminal RₐO- ou RₐR_{b}N- ; et
un ou deux de R₁ à R₅ représente(nt) des substituants sélectionnés dans le groupe constitué par R_{c}-Y-, R_{c}-Y-(CH₂)ₓ-, R_{c}-(CH₂)ₓ-Y-, R_{c}-Y-(CH₂)_{y}-O-, R_{c}-Y-(CH₂)_{y}-S- et R-Y-(CH₂)_{y}-NR₈- ; et les autres R₁ à R₅ représentent des atomes d'hydrogène ou des substituants sélectionnés dans le groupe constitué par les groupes alkyle en C₁-C₄, hydroxy, hydroxyalkyle en C₂-C₄, alcoxy en C₁-C₄, hydroxyalcoxy en C₂-C₄, halogène, amino et di(alkyle en C₁-C₄)amino ; ou
dans laquelle dans le mode de réalisation (2)
X représente un groupe terminal sélectionné dans le groupe constitué par R_{c}-Y₁-, R_{c}-(CH₂)ₓ-Y₁-, R_{c}-Y-(CH₂)_{y}-O- et R_{c}-Y-(CH₂)_{y}-NR₈- ; et
R₁ à R₅ représentent des atomes d'hydrogène ou des substituants sélectionnés dans le groupe constitué par les groupes alkyle en C₁-C₄, hydroxy, hydroxyalkyle en C₂-C₄, alcoxy en C₁-C₄, hydroxyalcoxy en C₂-C₄, halogène, amino et di(alkyle en C₁-C₄)amino ; ou
dans laquelle dans le mode de réalisation (3)
X représente un groupe terminal sélectionné dans le groupe constitué par R_{c}-Y₁-, R_{c}-(CH₂)ₓ-Y₁-, R_{c}-Y-(CH₂)_{y}-O- et R_{c}-Y-(CH₂)_{y}-NR₈- ; et
un ou deux de R₁ à R₅ représente(nt) des substituants sélectionnés dans le groupe constitué par R_{c}-Y-, R_{c}-Y-(CH₂)ₓ-, R_{c}-(CH₂)ₓ-Y-, R_{c}-Y-(CH₂)_{y}-O-, R_{c}-Y-(CH₂)_{y}-S- et R_{c}-Y-(CH₂)_{y}-NR₈- ; et les autres R₁ à R₅ représentent des atomes d'hydrogène ou des substituants sélectionnés dans le groupe constitué par les groupes alkyle en C₁-C₄, hydroxy, hydroxyalkyle en C₂-C₄, alcoxy en C₁-C₄, hydroxyalcoxy en C₂-C₄, halogène, amino et di(alkyle en C₁-C₄)amino ; et
dans laquelle dans les modes de réalisations (1), (2) et (3)
R₆ et R₇ représentent indépendamment l'un de l'autre un groupe alkyle en C₁-C₁₂, alcényle en C₂-C₈, cycloalkyle en C₅-C₈ ou phénylalkyle en C₁-C₃ ; ou R₆ et R₇ représentent ensemble un groupe alkylène en C₂-C₈, oxaalkylène en C₃-C₉ ou azaalkylène en C₃-C₉ ;
Rₐ et R_{b} représentent indépendamment l'un de l'autre un atome d'hydrogène, un groupe alkyle en C₁-C₁₂ ou alcényle en C₂-C₆ ; ou Rₐ et R_{b} représentent ensemble un groupe alkylène en C₄-C₆ et ensemble avec l'atome d'azote auquel ils sont liés forment un cycle de 5 ou 6 éléments, qui peut être interrompu par -O- ou par -N-R₈- ;
R_{c} représente une chaîne terminale linéaire ou ramifiée ZCF₂(-O-C₂F₄)ₚ-(CF₂)_{q}, dans laquelle Z représente H ou F ; un de p et q représente un nombre de 0 à 20 et l'autre est un nombre de 1 à 20 ;
x représente un nombre de 1 à 10 ;
y représente un nombre de 2 à 10 ;
Y représente un substituant bivalent sélectionné dans le groupe constitué par -S-, -NR₈-, -Si(R₉)₂-, -Si(R₉)₂-O-, -O-Si(R₉)₂-O- et -O-Si(R₉)₂-(CH₂)ₓ ;
Y₁ représente un substituant bivalent sélectionné dans le groupe constitué par -NR₈-, -O-Si(R₉)₂-, -O-Si(R₉)₂-O- et -O-Si(R₉)₂-(CH₂)ₓ ;
R₈ représente H ou un groupe alkyle en C₁-C₁₂ ; et
R₉ représente un groupe alkyle en C₁-C₁₂ ou phényle ; et
b) des composés polymérisables à insaturation éthylénique ; et facultativement, comme composants supplémentaires, un composé thermiquement réticulable ; et/ou d'autres additifs ; et/ou d'autres photoinitiateurs ;
est appliquée sur un support et durcie par irradiation avec une radiation électromagnétique d'une longueur d'onde de 200 nm jusqu'au domaine IR combinée à un durcissement thermique préalable, simultané ou ultérieur.

2. Procédé selon la revendication 1, **caractérisé en ce qu'**une composition comprenant
(a) au moins un initiateur de polymérisation de formule (I),
dans laquelle dans le mode de réalisation (1)
X représente un groupe terminal RₐO- ou RₐR_{b}N- ;
R₃, en position para du cycle phényle, représente un substituant sélectionné dans le groupe constitué par R_{c}-Y-, R_{c}-Y-(CH₂)ₓ-, R_{c}-(CH₂)ₓ-Y-, R_{c}-Y-(CH₂)_{y}-O- et R-Y-(CH₂)_{y}-S- ; et
R₁, R₂, R₄ et R₅ représentent des atomes d'hydrogène ou des substituants sélectionnés dans le groupe constitué par les groupes alkyle en C₁-C₄, hydroxy, hydroxyalkyle en C₂-C₄, alcoxy en C₁-C₄ et hydroxyalcoxy en C₂-C₄ ; ou
dans laquelle dans le mode de réalisation (2)
X représente un groupe terminal sélectionné dans le groupe constitué par R_{c}-Y₁-, R_{c}-(CH₂)ₓ-Y₁- et R_{c}-Y-(CH₂)_{y}-O- ;
R₁ à R₅ représentent des atomes d'hydrogène ou des substituants sélectionnés dans le groupe constitué par les groupes alkyle en C₁-C₄, hydroxy, hydroxyalkyle en C₂-C₄, alcoxy en C₁-C₄ et hydroxyalcoxy en C₂-C₄ ; ou
dans laquelle dans le mode de réalisation (3)
X représente un groupe terminal sélectionné dans le groupe constitué par R_{c}-Y₁-, R_{c}-(CH₂)ₓ-Y₁- et R-Y-(CH₂)_{y}-O- ;
R₃, en position para du cycle phényle, représente un substituant sélectionné dans le groupe constitué par R_{c}-Y-, R_{c}-Y-(CH₂)ₓ-, R_{c}-(CH₂)ₓ-Y-, R_{c}-Y-(CH₂)_{y}-O- et R_{c}-Y-(CH₂)_{y}-S- ; et
R₁, R₂, R₄ et R₅ représentent des atomes d'hydrogène ou des substituants sélectionnés dans le groupe constitué par les groupes alkyle en C₁-C₄, hydroxy, hydroxyalkyle en C₂-C₄, alcoxy en C₁-C₄ et hydroxyalcoxy en C₂-C₄ ; et dans laquelle dans les modes de réalisations (1), (2) et (3)
R₆, R₇, Rₐ, R_{b}, R_{c}, x, y, Y et Y₁ sont comme défini dans la revendication 1 ; et
b) des composés polymérisables à insaturation éthylénique ; et facultativement, comme composants supplémentaires, un composé thermiquement réticulable ; et/ou d'autres additifs ; et/ou d'autres photoinitiateurs ;
est appliquée sur un support et durcie comme défini dans la revendication 1.

3. Procédé selon la revendication 1, **caractérisé en ce qu'**une composition comprenant
(a) au moins un initiateur de polymérisation de formule (I),
dans laquelle dans le mode de réalisation (1)
X représente un groupe terminal RₐO- ou RₐR_{b}N- ; et
R₃, en position para du cycle phényle, représente un substituant sélectionné dans le groupe constitué par R_{c}-(CH₂)ₓ-Y- et R_{c}-Y-(CH₂)_{y}-O- ; et
les autres, R₁, R₂, R₄ et R₅, représentent des atomes d'hydrogène ; ou
dans laquelle dans le mode de réalisation (2)
X représente un groupe terminal R_{c}-Y₁- ; et
R₁ à R₅ représentent des atomes d'hydrogène ; ou
dans laquelle dans le mode de réalisation (3)
X représente un groupe terminal R_{c}-Y₁- ; et
R₃, en position para du cycle phényle, représente un substituant R_{c}-Y-(CH₂)_{y}-O- ; et
les autres, R₁, R₂, R₄ et R₅, représentent des atomes d'hydrogène ; et
dans laquelle dans les modes de réalisations (1), (2) et (3)
R₆ et R₇ représentent indépendamment l'un de l'autre un groupe alkyle en C₁-C₁₂ ou benzyle ;
Rₐ et R_{b} représentent indépendamment l'un de l'autre un atome d'hydrogène ou un groupe alkyle en C₁-C₁₂ ; ou Rₐ et R_{b} ensemble avec l'atome d'azote auquel ils sont liés forment un cycle morpholinyle, pipéridinyle ou pipérazinyle ;
R_{c} représente une chaîne terminale linéaire ou ramifiée ZCF₂(-O-C₂F₄)ₚ-(CF₂)_{q}, dans laquelle Z représente H ou F ; un de p et q représente un nombre de 0 à 20 et l'autre est un nombre de 1 à 20 ;
x représente un nombre de 1 à 10 ;
y représente un nombre de 2 à 10 ;
Y et Y₁ représentent indépendamment l'un de l'autre un substituant bivalent sélectionné dans le groupe constitué par -S- et -O-Si₁(R₉)₂-(CH₂)ₓ ;
R₉ représente un groupe alkyle en C₁-C₈ ou phényle ; et
b) des composés polymérisables à insaturation éthylénique ; et facultativement, comme composants supplémentaires, un composé thermiquement réticulable ; et/ou d'autres additifs ; et/ou d'autres photoinitiateurs ;
est appliquée sur un support et durcie comme défini dans la revendication 1.

4. Procédé selon la revendication 1, **caractérisé en ce qu'**une composition comprenant
(a) au moins un initiateur de polymérisation de formule (I') 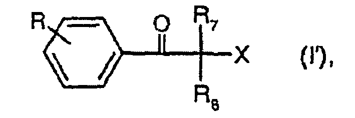 dans laquelle dans le mode de réalisation (1)
X représente un groupe terminal RₐO- ou RₐR_{b}N- ; et
R représente un substituant sélectionné dans le groupe constitué par R_{c}-Y-, R_{c}-Y-(CH₂)ₓ-, R_{c}-(CH₂)ₓ-Y-, R_{c}-Y-(CH₂)_{y}-O-, R_{c}-Y-(CH₂)_{y}-S- et R_{c}-Y-(CH₂)_{y}-NR₈- ; ou
dans laquelle dans le mode de réalisation (2)
X représente un groupe terminal sélectionné dans le groupe constitué par R_{c}-Y₁-, R_{c}-(CH₂)ₓ-Y₁- et R_{c}-Y-(CH₂)_{y}-O- ; et
R représente un atome d'hydrogène ou un substituant sélectionné dans le groupe constitué par les groupes alkyle en C₁-C₄, hydroxy, hydroxyalkyle en C₂-C₄, alcoxy en C₁-C₄ et hydroxyalcoxy en C₂-C₄ ; ou
dans laquelle dans le mode de réalisation (3)
X représente un groupe terminal sélectionné dans le groupe constitué par R_{c}-Y₁-, R_{c}-(CH₂)ₓ-Y₁- et R_{c}-Y-(CH₂)_{y}-O- ; et
R représente un substituant sélectionné dans le groupe constitué par R_{c}-Y-, R_{c}-Y-(CH₂)ₓ-, R_{c}-(CH₂)ₓ-Y-, R_{c}-Y-(CH₂)_{y}-O-, R_{c}-Y-(CH₂)_{y}-S- et R_{c}-Y-(CH₂)_{y}-NR₈- ; et
dans laquelle dans les modes de réalisations (1), (2) et (3)
R₆, R₇, R₈ et R₉ sont comme défini dans la revendication 1 ;
Rₐ, R_{b} et R_{c} sont comme défini dans la revendication 1 ;
x et y sont comme défini dans la revendication 1 ;
Y et Y₁ sont comme défini dans la revendication 1 ; et
b) des composés polymérisables à insaturation éthylénique ; et facultativement, comme composants supplémentaires, un composé thermiquement réticulable ; et/ou d'autres additifs ; et/ou d'autres photoinitiateurs ;
est appliquée sur un support et durcie comme défini dans la revendication 1.

5. Procédé selon la revendication 4, **caractérisé en ce qu'**une composition comprenant
a) au moins un initiateur de polymérisation de formule (I') comme défini dans la revendication 4 ; et
b) des composés polymérisables à insaturation éthylénique ; et facultativement, d'autres additifs ; et/ou d'autres photoinitiateurs ;
est appliquée sur un support et durcie par irradiation avec une radiation électromagnétique d'une longueur d'onde de 200 nm jusqu'au domaine IR.

6. Procédé selon la revendication 1, **caractérisé en ce que** la composition comprend comme composant supplémentaire
c) au moins un composé thermiquement réticulable contenant au moins une double liaison ;
et le durcissement est réalisé par irradiation avec une radiation électromagnétique d'une longueur d'onde de 200 nm jusqu'au domaine IR combinée à un durcissement thermique préalable, simultané ou ultérieur.

7. Procédé selon la revendication 6, **caractérisé en ce que** le composé thermiquement réticulable c) est un liant basé sur une composition d'un polyacrylate avec de la mélamine ou un dérivé de la mélamine, ou une composition basée sur un polyacrylate polyol et un polyester polyol avec un polyisocyanate ou un polyisocyanurate non bloqué ou un polyester polyol avec un polyester polyol avec un polyisocyanate ou un polyisocyanurate non bloqué.

8. Procédé selon la revendication 1, pour la préparation de revêtements de surface pigmentés et non pigmentés, de revêtements en poudre, de composites et de revêtement de câbles en fibre de verre.

9. Substrat enduit qui est enduit sur au moins une surface avec une composition selon la revendication 1.

10. Initiateur de polymérisation de formule (I) tel que décrit dans la revendication 1.

11. Initiateur de polymérisation de formule (I) selon la revendication 10, sélectionné dans le groupe constitué par 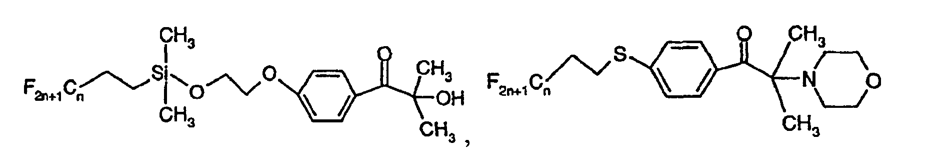 et, 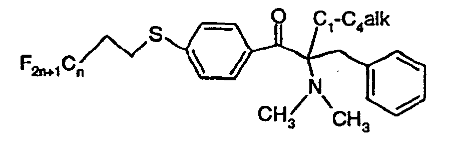 où n représente un nombre de 3 à 20.

12. Initiateur de polymérisation de formule (I) selon la revendication 10, sélectionné dans le groupe constitué par

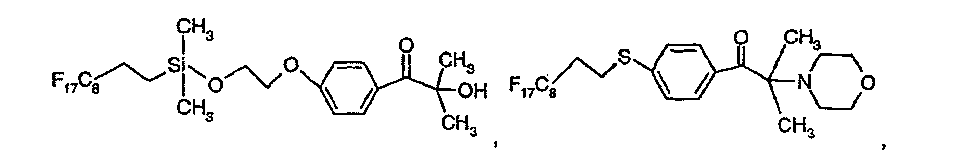

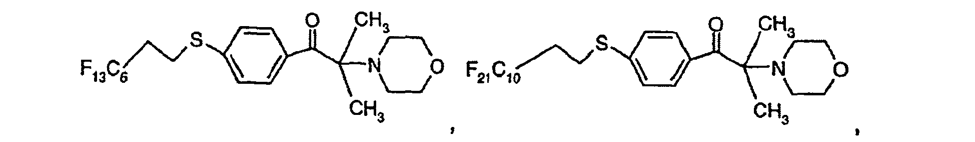

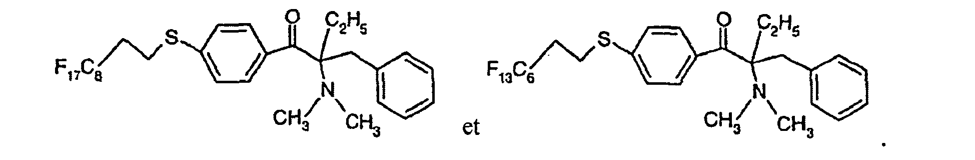

13. Composition comprenant
a) au moins un initiateur de polymérisation de formule (I') selon la revendication 4 ; et
b) des composés polymérisables à insaturation éthylénique.

14. Composition selon la revendication 13, comprenant en outre des composants sélectionnés dans le groupe constitué par
c) au moins un composé thermiquement réticulable ;
d) d'autres additifs ; et
e) d'autres photoinitiateurs.
